# EUROPÄISCHE PATENTSCHRIFT

(11) **EP 1 563 822 B1**
(45) Veröffentlichungstag und Bekanntmachung des Hinweises auf die Patenterteilung: **02.09.2020**
(21) Anmeldenummer: 05003089.9
(22) Anmeldetag: 14.02.2005
(51) Int. Cl.: A61K 6/90, A61K 6/896

(54) **Dentalmaterial auf Basis von alkoxysilylfunktionellen Polyethern mit einem Katalysator**
Dental material based on alkoxysilyl functional polyether comprising a catalyst
Matériau dentaire à base de polyéthers fonctionnels alkoxysilyles comprenant un catalysateur

(30) Priorität: 13.02.2004 DE 102004008022
(43) Veröffentlichungstag der Anmeldung: 17.08.2005
(73) Patentinhaber: Kettenbach GmbH & CO. KG, 35713 Eschenburg (DE)
(72) Erfinder: Bublewitz, Alexander,, 35745 Herborn (DE); Reber, Jens-Peter, 58540 Meinerzhagen (DE)
(74) Vertreter: Keil & Schaafhausen Patentanwälte PartGmbB

(56) Entgegenhaltungen:
- EP-A- 0 269 819
- EP-A- 1 081 191
- EP-A- 1 226 808
- EP-A- 1 402 873
- EP-A1- 0 902 042
- EP-B1- 0 544 674
- WO-A-99/48942
- WO-A-2004/052994
- US-A- 4 375 549
- US-A- 5 086 148

## Beschreibung

Die vorliegende Erfindung betrifft kondensationsvernetzende Dentalmaterialien, insbesondere kondensationsvernetzende Zweikomponenten-Dentalabformmaterialien, auf Basis von alkoxysilylfunktionellen Polyethern, welche insbesondere zur Abdrucknahme geeignet sind, und deren Verwendung. Derartige Materialien werden in der Dentalmedizin bspw. zur Zahnabdrucknahme, Bissregistrierung, Zahnprothesenunterfütterung, als provisorischer und permanenter Dentalzement, provisorisches Verschlussmaterial oder dentales Zahntechnik-Dubliermaterial eingesetzt.

Bekannte kondensationsvernetzende Dentalmaterialien enthalten üblicherweise hydroxylfunktionelle Polymere mit einem Siliconrückgrat, welche in Gegenwart organischer Zinnverbindungen als Katalysatoren, Alkoxysilanen und/oder Kieselsäurestern als Vernetzer und Wasser aushärten. Allerdings sind derartige Materialien aufgrund des Siliconrückgrats der Polymere vergleichsweise hydrophob, so dass diesen zwecks Herabsetzung der Oberflächenspannung und zur Einstellung der erforderlichen Benetzbarkeit erhebliche Anteile an Tensiden zugefügt werden müssen. Ein weiterer Nachteil dieser Zusammensetzungen liegt in dem Einsatz toxikologisch bedenklicher organischer Zinnverbindungen als Katalysator.

Alternativ dazu sind Zweikomponenten-Dentalmaterialien bekannt, welche terminale Alkoxysilylgruppen aufweisende Polymere mit einem hydrophilen Polyetherrückgrat enthalten, die zur Benetzung der feuchten Zahnsubstanz ausreichend hydrophile Eigenschaften aufweisen. Üblicherweise bestehen diese Materialien aus einer Basiskomponente enthaltend alkoxysilylfunktionelle Polyether mit einem mittleren Molekulargewicht von 800 bis 20.000 g/mol, welche synthesebedingt auch Harnstoff- und/oder Urethangruppen aufweisen können, Füllstoffe sowie ggf. weitere Zusatzstoffe, und einer Katalysatorkomponente, welche neben Füll- und ggf. weiteren Hilfsstoffen eine organische und/oder anorganische Säure als Katalysator enthält.

Aus der EP 0 269 819 B1 sind kondensationsvernetzende Zweikomponenten-Dentalmaterialien bekannt, deren Basiskomponente Alkoxysilylendgruppen enthaltende Polyadditionsprodukte mit einer überwiegend linearen Molekülstruktur und einem mittleren Molekulargewicht von 800 bis 20.000 g/mol enthalten, welche einen Gehalt an Polyethergruppen von 25 bis 90 Gew.-%, einen Gehalt an Urethangruppen von 0,5 bis 10 Gew.-%, einen Gehalt an Harnstoffgruppen von 0,5 bis 10 Gew.-% sowie einen Gehalt an terminalen Alkoxysilylgruppen von 1 bis 25 Gew.-%, und, deren Katalysatorkomponente eine Abmischung enthaltend Wasser sowie organische und/oder anorganische Säuren in Gewichtsmengenverhältnissen (Wasser/Säure) von 1:0,01 bis 1:40, aufweisen. Allerdings ist die Synthese der in der Basiskomponente enthaltenden funktionellen Polyetherpolymere sehr aufwendig und teuer. Ein weiterer Nachteil dieser Dentalmaterialien liegt in dem Einsatz säurehaltiger Katalysatoren. Zum einen können durch die Säurekatalysatoren bei der Abformung im Patientenmund die Mundschleimhaut und der Zahnschmelz durch Säureätzung geschädigt werden. Zudem erlauben diese Systeme keinen Zusatz an stickstoffbasenhaltigen Substanzen, wie Adstringentien, bspw. Epinephrine, oder anderen säurelabilen therapeutischen Zusätze, da diese durch den Säurekatalysator aufgrund von Protonierung oder Spaltung inaktiviert werden. Ferner erfordert der Einsatz von Säuren bei der Produktion der Dentalmaterialien entsprechende Sicherheitsvorkehrungen.

In der EP 1 226 808 A2 werden kondensationsvernetzende Zweikomponenten-Dentalmaterialien bestehend aus einer Basis- und Katalysatorkomponente offenbart, deren Basiskomponente alkoxysilylfunktionelle Polyether mit linearer oder verzweigter Hauptkette und einem mittleren Molekulargewicht von 800 bis 20.000 g/mol enthalten, welche einen Gehalt an Polyethergruppen von 20 bis 95 Gew.-%, einen Gehalt an terminalen Alkoxysilylgruppen von 0,2 bis 25 Gew.-% sowie ggf. einen Gehalt an Urethangruppen oder Harnstoffgruppen von bis zu 10 Gew.-%, und, deren Katalysatorkomponente eine Mischung enthaltend Wasser sowie organische und/oder anorganische Säuren in Gewichtsmengenverhältnissen von 1:0,01 bis 1:40, aufweisen. Vorzugsweise enthält die Katalysatorkomponente p-Toluolsulfonsäurehydrat als Katalysator sowie ein Polyetherdiol und weitere Zusatzstoffe, wie Füllstoffe, Paraffin, Emulgator und dergl. Zwar sind die in diesen Dentalmaterialien eingesetzten funktionellen Polyetherpolymere gegenüber den zuvor genannten einfacher und kostengünstiger zu synthetisieren und zeichnen sich durch eine bessere Abbindekinetik aus. Allerdings machen auch diese Dentalmaterialien von säurehaltigen Katalysatoren Gebrauch, so dass einerseits die Gefahr der Schädigung der Mundschleimhaut sowie des Zahnschmelzes bei der Abformung im Patientenmund besteht und diesen Materialien zudem keine säurelabilen therapeutischen Zusätze zugesetzt werden können. Ein weiterer Nachteil der Systeme liegt in deren geringeren Lagerstabilität. Eine unabhängig von der Lagerungszeit gleichbleibende Abbindezeit ist allerdings eine der wichtigsten Anforderungen an ein dentales Abformmaterial.

In der WO 99/48942 wird vorgeschlagen, zur Vernetzung von als Kleb- oder Dichtstoffen einzusetzenden und Polyethergruppen aufweisenden Polyurethanen metalllorganische Verbindungen, wie Eisen- oder Zinnverbindungen, bspw. Zinn(II)oktat, oder tertiäre Amine, wie Triethylamin, einzusetzen. Ein Nachteil dieser Katalysatoren liegt jedoch, insbesondere was die schwermetallorganischen Verbindungen anbelangt, in deren hohen Toxizität, so dass bei der Produktion der Materialien geeignete sicherheitstechnische Vorkehrungen zu treffen sind und eine Verwendung der Materialien als Dentalabformmassen nicht ohne weiteres möglich ist. Zudem sind die in diesen Materialien eingesetzten Polyurethane aufgrund deren hohen Anteil an Urethangruppen pro Molekül grund deren hohen Anteil an Urethangruppen pro Molekül durch starke intermolekulare Wechselwirkungen gekennzeichnet, was bei gegebener Kettenlänge der Moleküle zu einer verglichen mit alkoxysilylfunktionellen Polyethern, die pro Molekül nur zwei Urethangruppen aufweisen, zu einer erhöhten Viskosität der Materialien führt, weswegen in diesen Materialien weniger Füllstoffe eingesetzt werden können, was wiederum erhöhte Herstellungskosten bedingt.

In der WO 96/38453 werden als Dichtmassen, Klebstoffe oder Beschichtungen mit hoher Klebkraft einsetzbare Ein- und Zweikomponentenmaterialien auf Basis von alkoxysilylfunktionellen Polyurethanen offenbart, welche als Katalysator Metallsalze, organische Säuren, anorganische Säuren oder Amine enthalten können.

Aus der DD 83 248 sind Härter für Organopolysiloxane bestehend aus Umsetzungsprodukten spezieller Organozinnverbindungen und spezieller monomerer Siliziumverbindungen, ein Herstellungsverfahren für diese Katalysatoren sowie deren Anwendung für die Kaltvernetzung von Organopolysiloxanen bekannt. Diese Druckschrift lehrt, dass die Verwendung von Aminen oder Siliziumverbindungen mit Si-C-gebundenen Amino-Alkylestern als Katalysatoren den aushärtenden Elastomeren Hafteigenschaften verleiht, weswegen der Einsatz von Aminen als Katalysator in kondensationsvernetzenden Dental- und Abformmaterialien, die im ausgehärteten Zustand gerade keine Klebrigkeit aufweisen dürfen, unmöglich ist.

Aus der US 4,375,549 A1 beschreibt ein chemisches Verfahren zur Herstellung von organischen Silanen mit Alkoxy- oder Aryloxysubstituenten, auch als organische Silylether bezeichnet. Solche Ether haben unterschiedliche Verwendungszwecke. Monomere Ether dieser Art sind wertvolle Funktionsflüssigkeiten, während polymere Ether, die nach diesem Verfahren herstellbar sind, nützliche formbare Kunststoffe sind.

Die EP 0 902 042 A1 bezieht sich auf eine einer härtbaren Zusammensetzung. Diese enthält (a) ein Oxyalkylenpolymer, das mindestens eine reaktive Silylgruppe pro Molekül beinhaltet, und (b) eine Verbindung mit einer intramolekularen α, β-diol oder α, γ-diol-Struktur.

Dokument EP 0 544 674 B1 befasst sich mit Organo(poly)siloxanmassen, die unter Ausschluss von Wasser lagerfähig sind, bei Beimischung von Wasser jedoch schon bei Raumtemperatur unter Abspaltung von Alkoholen zu Elastomeren vernetzt werden können. Dazu sind die Organo(poly)siloxane definiert als Verbidnungen mit Organyloxy- und Hydrogengruppen aufweisenden, endständigen Siloxaneinheiten.

US 4375549 A1 offenbart die Synthese von organischen Silylethern, welche aus der Reaktion von Carbonaten mit organischen Silylhaliden in Gegenwart eines Initiators in einem Temperaturbereich von 50 °C bis 250 °C. Kronenether können eingesetzt werden, um die Löslichkeit der Initiatorverbindung zu erhöhen. Als einsetzbare Initiatorverbindungen werden u.a. organische Lewis- und Mineralsäuren, sowie Basen aus der Gruppe der Amine, Alkalimetallhydroxiden und -carbonaten, sowie Nitrat-, Halogenid- und Sulfatsalze der Alkalimetalle und Gruppe Ib, IIb oder VII Metalle des PSE angeführt.

EP 0 902 042 A1 offenbart härtbare Massen für den Einsatz als Dichtmittel für die Bauindustrie, welche nach Kondensation eine gummiartige Beschaffenheit ohne dabei klebrig zu sein oder. Als generell mögliche Katalysatoren (siehe Absatz 40) werden Organometallverbindungen u.a. der Elemente Titan, Zinn, Aluminium und Blei aufgeführt, als auch primäre, sekundäre, tertiäre aliphatische und aromatische Amine, sowie einige heterozyklische Stickstoffverbindungen, wie 1,8-Diazabicyclo(5.4.0)undec-7-en (DBU).

EP 0 544 674 B1 offenbart Organopolysiloxanmassen, welche vorwiegend in Gegenwart von Katalysatoren der 8. Nebengruppe des Periodensystems und deren anorganischen und organischen Verbindungen kondensationsvernetzt werden. Zu den gelisteten Füllstoffen, mit welchen verhärtende Massen erhalten werden, zählen u.a. anorganische Füllstoffe mit Oberflächen größer und/oder kleiner 50 m²/g, Diatomeenerde, Pigmente und Ruß. Die härtbaren Massen eignen sich für den Einsatz als Dichtmaterialien u.a. in Luft- und Wasserfahrzeugen oder Gebäuden.

Nach dem Stand der Technik können somit Amine als Katalysator für kondensationsvernetzende Klebstoffe, Beschichtungen und Dichtmassen, in denen eine Klebrigkeit der ausgehärteten Materialien erwünscht ist, eingesetzt werden, hingegen nicht in Dentalmaterialien, da Amine den nach der Aushärtung resultierenden Elastomeren Hafteigenschaften, die bei Dentalmaterialien, insbesondere Dentalabformmaterialien, unerwünscht sind, verleihen.

Aufgabe der vorliegenden Erfindung ist es daher, ein hydrophiles kondensationsvernetzendes Dentalmaterial, insbesondere ein kondensationsvernetzendes Zweikomponenten-Dentalabformmaterial, auf Basis von Alkoxysilylpolyethern zur Verfügung zu stellen, welches lagerstabil ist, eine gute Biokompatibilität aufweist, den Zusatz säurelabiler Zusätze, wie stickstoffhaltiger Adstringentien, Medikamente, Bakterizide, Fungizide und dergl. ermöglicht und toxikologisch möglichst unbedenkliche Inhaltsstoffe aufweist, ohne nach dessen Aushärtung Hafteigenschaften zur Mundschleimhaut und zur Zahnsubstanz aufzuweisen.

Erfindungsgemäß wird diese Aufgabe durch ein kondensationsvernetzendes Dentalmaterial der Zusammensetzung gemäß Patentanspruch 1 gelöst.

Überraschenderweise konnte im Rahmen der vorliegenden Erfindung gefunden werden, dass kondensationsvernetzende Zweikomponenten-Dentalmaterialien auf Basis von Alkoxysilylpolyethern enthaltend verstärkende Füllstoffe und/oder nichtverstärkende Füllstoffe sowie ggf. weitere in Dentalmaterialien übliche Zusatzstoffe durch die erfindungsgemäß einzusetzenden organischen Basen mit einer für Dentalmaterialien geeigneten Reaktionskinetik, insbesondere einer hierfür ausreichenden Verarbeitungs- und Abbindezeit, vernetzt und ausgehärtet werden können, ohne dass die ausgehärteten Materialien klebrig sind bzw. elastomere Hafteigenschaften zur Mundschleimhaut und zur Zahnsubstanz aufweisen. Insbesondere zeichnen sich die erfindungsgemäßen Dentalmaterialien durch eine hervorragende Lagerstabilität aus. Ein weiterer Vorteil der erfindungsgemäßen Dentalmaterialien liegt darin, dass diesen auch säurelabile Zusätze, insbesondere stickstoffhaltige Adstringentien, Medikamente, Bakterizide, Fungizide und dergl., zugesetzt werden können, ohne dass diese während der Lagerung abgebaut werden.

Die erfindungsgemäßen Dentalmaterialien können sowohl als Einkomponentenals auch als Zweikomponentenmaterial formuliert sein. Während die Formulierung der Einkomponenten-Dentalmaterialien möglichst absolut wasserfrei sein muss, um eine Reaktion der alkoxysilylfunktionellen Polyether während der Lagerung zu verhindern, und die Reaktion der alkoxysilylfunktionellen Polyether nach der Applikation der Materialien auf dem abzuformenden Gegenstand durch Luftfeuchtigkeit initiiert wird, ist der Katalysatorkomponente des erfindungsgemäßen Zweikomponenten-Dentalmaterials vorzugsweise Wasser zugesetzt. Vorzugsweise werden die erfindungsgemäßen Zweikomponenten-Dentalmaterialien so formuliert, dass die

### Basiskomponente A

a) wenigstens einen alkoxysilylfunktionellen Polyether,
b) wenigstens einen verstärkenden Füllstoff b₁) mit einer BET-Oberfläche von mindestens 50 m²/g und/oder wenigstens einen nichtverstärkenden Füllstoff b₂) mit einer BET-Oberfläche von weniger als 50 m²/g,
und die Katalysatorkomponente B
c) Wasser und
d) wenigstens einen Katalysator
enthält, wobei der wenigstens eine Katalysator eine organische Base mit einem in Acetonitril gemessenen pk_{BH+}-Wert von mindestens 20 ist.

Unabhängig von der Formulierung als Ein- oder Zweikomponentenmaterial weist die erfindungsgemäß als Katalysator einzusetzende wenigstens eine Base vorzugsweise einen in Acetonitril gemessenen pK_{BH+}-Wert von mindestens 21, besonders bevorzugt einen in Acetonitril gemessenen pK_{BH+}-Wert von mindestens 21, besonders bevorzugt von mindestens 22 und ganz besonders bevorzugt von mindestens 23 auf.

Gemäß der vorliegenden Erfindung wird als Katalysator d) eine organische Base eingesetzt, welche wenigstens eine Struktureinheit gemäß der allgemeinen Formel I 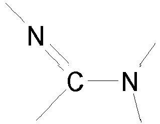 und/oder gemäß der allgemeinen Formel II und/oder gemäß der allgemeinen Formel III 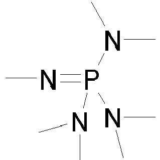 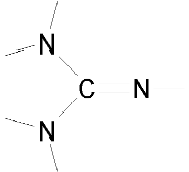 umfasst.

Insbesondere werden gute Ergebnisse erzielt, wenn der Katalysator d) wenigstens eine aus der aus 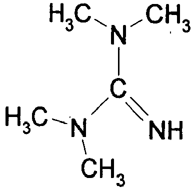 1,1,3,3-Tetramethylguanidin (TMG) 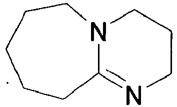 Diazabicyclo[5.4.0]undec-7-en (DBU), 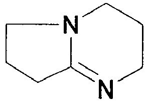 1,5-Diazabicyclo[4.3.0]non-5-en (DBN) 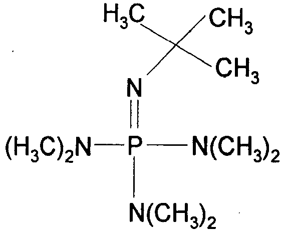 tert-Butylimino-tris(dimethylamino)phosphoran 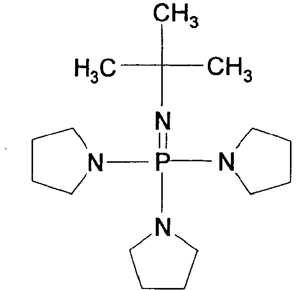 tert-Butylimino-tri(pyrrolidino)phosphoran 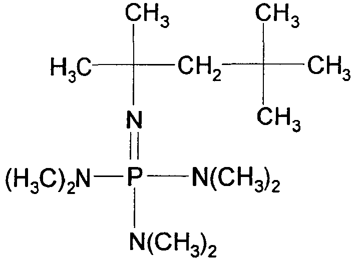 tert-Octylimino-tris(dimethylamino)phosphoran 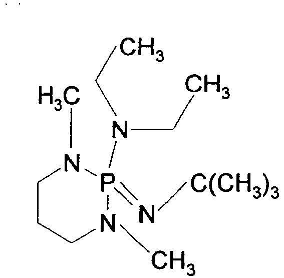 2-tert-Butylimino-2-diethylamino-1,3-dimethyl-perhydro- 1,3,2-diazaphosphorin 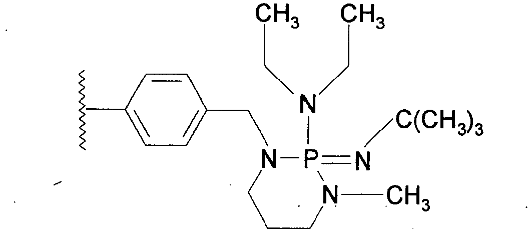 2-tert-Butylimino-2-diethylamino-1,3-dimethyl-perhydro- 1,3,2-diazaphosphorin auf Polystyrol 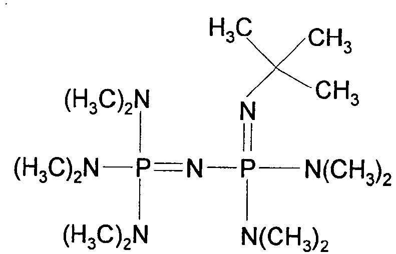 1-tert-Butyl-2,2,4,4,4-pentakis(diethylamino)-2Λ5, 4A5-catenadi(phosphazen) 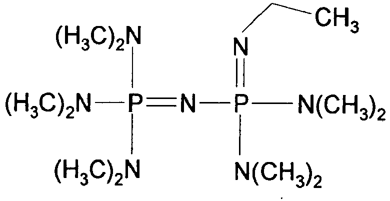 1-Ethyl-2,2,4,4,4-pentakis(diethylamino)-2Λ5, 4Λ5-catenadi(phosphazen) 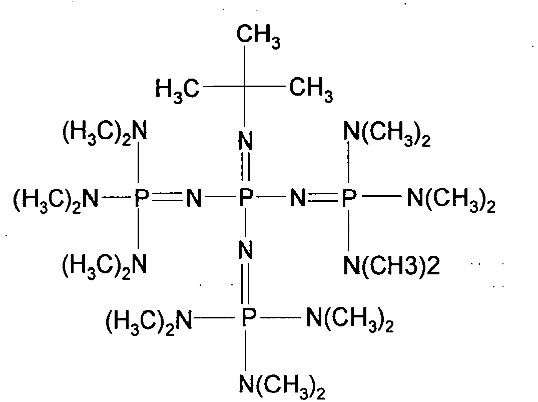 1-tert-Butyl-4,4,4-tris(dimethylamino)-2,2-bis[tris(dimethylamino)-phosphor-anylidenamino]-2Λ⁵,4Λ⁵-catenadi(phosphazen) 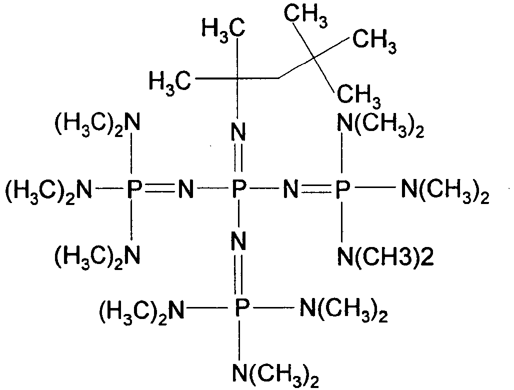 1-tert-Octyl-4,4,4-tris(dimethylamino)-2,2-bis[tris(dimethylamino)-phosphor-anylidenamino]-2Λ⁵,4Λ⁵-catenadi(phosphazen) 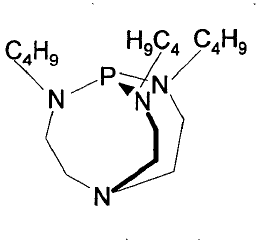 2,8,9-Triisobutyl-2,5,8,9-tetraaza-1-phosphabicyclo[3.3.3]undecan 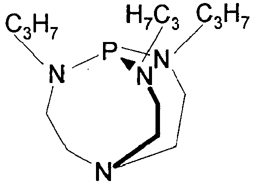 2,8,9-Triisopropyl-2,5,8,9-tetraaza-1-phosphabicyclo[3.3.3]undecan 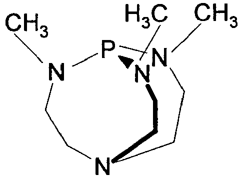 2,8,9-Trimethyl-2,5,8,9-tetraaza-1-phosphabicyclo[3.3.3]undecan 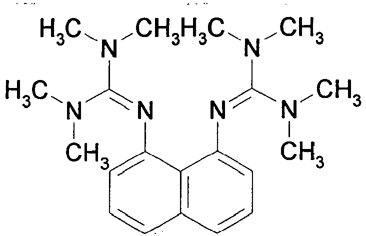 1,8-Bis(tetramethylguanidino)naphthalen (TMGN)
sowie 2-tert-Butyl-1,1,3,3-tetramethylguanidin, 1,5,7-Triazabicyclo(4.4.0)dec-5-en, 7-Methyl-1,5,7-triazabicyclo(4.4.0)dec-5-en, 1,5-Diazabicyclo[4.3.0)non-5-en und 3,3,6,9,9-Pentamethyl-2,10-diazabicyclo-(4.4.0)dec-1-en bestehenden Gruppe ausgewählte organische Base ist.

Ganz besonders bevorzugt enthält das erfindungsgemäße Dentalmaterial als Katalysator wenigstens eine aus der aus tert-Butylimino-tri(pyrrolidino)phosphoran, 1-tert-Butyl-2,2,4,4,4-pentakis(diethylamino)-2Λ5, 4A5-catenadi(phosphazen), 1-tert-Butyl-4,4,4-tris(dimethylamino)-2,2-bis[tris(dimethylamino)-phosphoranylidenamino]-2Λ⁵,4Λ⁵-catenadi(phosphazen), tert-Octylimino-tris(dimethylamino)phosphoran, 2,8,9-Tri-isopropyl-2,5,8,9-tetraaza-1-phosphabicyclo[3.3.3]undecan, 1,5-Diazabicyclo[4.3.0]non-5-en, 1,1,3,3-Tetramethylguanidin, Diazabicyclo[5.4.0]undec-7-en, 7-Methyl-1,5,7-triazabicyclo(4.4.0)dec-5-en, 2-tert-Butyl-1,1,3,3-tetra-methylguanidin, 1,5,7-Triazabicyclo(4.4.0)dec-5-en und 1,8-Bis(tetramethylguanidino)naphthalen bestehenden Gruppe ausgewählte Base.

In Weiterbildung des Erfindungsgedankens wird vorgeschlagen, dass die vorgenannten, erfindungsgemäß als Katalysator einzusetzenden organischen Basen Alkoxysilylgruppen aufweisen. Dadurch wird erreicht, dass die Base nach Aushärten des Dentalmaterials in der Polyethermatrix eingebunden ist und aus dem dentalen Abformmaterial nicht mehr herausgelöst werden kann.

Erfindungsgemäß können die Dentalmaterialien als Katalysator d) eine oder, in beliebiger Kombination miteinander, mehrere der zuvor genannten organischen Basen enthalten. Vorzugsweise enthält das Dentalmaterial nur eine der zuvor genannten organischen Basen als Katalysator d). Besonders bevorzugt werden neben der einen oder mehreren erfindungsgemäß einzusetzenden organischen Basen keine weiteren Katalysatoren, insbesondere keine metällorganischen Metallsalze, keine Amine mit pk_{BH+}-Werten < 20 und keine freien Säuren, eingesetzt.

Wie der Fachmann erkennt hängt die Menge an einzusetzender Katalysatorbase u.a. von der Löslichkeit der Base in der eingesetzten Polyethermatrix ab. Vorzugsweise beträgt die Menge der Katalysatorbase(n), bezogen auf die Gesamtmischung des Dentalmaterials, 0,001 bis 1 mmol/g, bevorzugt 0,001 bis 0,5 mmol/g, ganz besonders bevorzugt 0,001 bis 0,1 mmol/g und höchst bevorzugt 0,005 bis 0,05 mmol/g. Selbstverständlich muss die als Katalysator d) eingesetzte organische Base bzw. die Basenmischung eine Mindestlöslichkeit in der eingesetzten Polyethermatrix aufweisen, um überhaupt katalytisch wirken zu können.

Um die Menge an einzusetzender Katalysatorbase möglichst gering zu halten, wird in Weiterbildung des Erfindungsgedankens vorgeschlagen, in dem Dentalmaterial eine Base mit einer hinreichend hohen Löslichkeit in dem Polyethermaterial, d.h. mit einer ausreichenden katalytischen Aktivität, einzusetzen, wobei die katalytische Aktivität im Sinne der vorliegenden Patentanmeldung durch die Aushärtezeit nach ISO 4823 (Ausgabe 1992), bestimmt durch Rückstellung nach der Verformung, charakterisiert ist. Vorzugsweise wird eine Base eingesetzt, die mit Polytetrahydrofuran, Polyethylenglycol und besonders bevorzugt Polypropylenglycol sowie deren Mischungen und Copolymerisaten als Polyethermatrix eine Aushärtezeit kleiner gleich 30 min, bevorzugt kleiner gleich 15 min für eine dentale Zahntechnik-Dubliermasse bzw. eine Aushärtezeit kleiner gleich 15 min, bevorzugt kleiner gleich 10 min, besonders bevorzugt kleiner gleich 7 min und höchst bevorzugt kleiner gleich 6 min für eine dentale Abformmasse ergibt.

Wenn das erfindungsgemäße Dentalmaterial als Einkomponentenmaterial formuliert ist, sollte es möglichst absolut wasserfrei sein, um eine Reaktion der alkoxysilylfunktionellen Polyether während der Lagerung zu vermeiden.

In dem Fall, dass das Dentalmaterial als Zweikomponentenmaterial formuliert ist, enthält die Katalysatorkomponente B vorzugsweise Wasser, wohingegen die Basiskomponente A möglichst absolut wasserfrei ist. Vorzugsweise enthält die Katalysatorkomponente B des erfindungsgemäßen Zweikomponenten-Dentalmaterials, bezogen auf die Gesamtmischung, 0,005 bis 3 mmol/g, besonders bevorzugt 0,01 bis 2 mmol/g und ganz besonders bevorzugt 0,02 bis 1 mmol/g an Wasser.

Dem erfindungsgemäßen Dentalmaterial können optional Puffersubstanzen e) zugesetzt sein, um den pH-Wert der Katalysatorkomponente B und der Basiskomponente A einzustellen. Im Rahmen der vorliegenden Erfindung können als Puffersubstanzen insbesondere alle Salze aus schwachen Säuren und starken Basen bzw. aus schwachen Basen und starken Säuren eingesetzt werden. Als besonders geeignet zu diesem Zweck haben sich insbesondere Puffersysteme mit wenigstens einer aus der aus Alkalimetallhydrogencarbonat, Dialkalihydrogenphosphat, Tris(hydroxymethyl)aminomethan, Phthalsäure-Monoalkalisalz, Phthalsäuremonotetramethylammoniumsalz, Ammoniumsalzen von Aminen, cyclischen Aminen, Amiden und cyclischen Amiden, N-(2-Acetamido)-2-aminoethansulfonsäure, N-(2-Acetamido)iminodiessigsäure, ß-Alanin, Ameisensäure (98 - 100%), 2-Amino-2-methyl-1-propanol, Barbital, Barbital-Natrium, Bernsteinsäure, N,N-Bis-(2-hydroxyethyl)-2-aminoethansulfonsäure, N,N-Bis(2-hydroxyethyl)-glycin, 2,2-Bis-(Hydroxyethyl)-(iminotris)-(hydroxyamethyl)methan, Borsäure, CHAPS (3-[(3-Cholamidoproypl)-dimethyl-ammonio]-propan-sulfonat), Citronensäure-Monohydrat, 2-(Cyclo-hexylamino)-ethansulfonsäure, Diethanolamin, Essigsäure (Eisessig; 100 %), Glycin, Glycylglycin, 2-[4-2-Hydroxyethyl)-1-piperazinyl]-ethansulfonsäure, 2-[4-(2-Hydroxyethyl)-1-piperazinyl]-ethansulfonsäure Natriumsalz, 3-[4-(2-Hydroxyethy))-1-piperazinyl]-propansuifonsäure, Imidazol, Kaliumdihydrogenphosphat, di-Kaliumhydrogenphosphat-Trihydrat, Kaliumhydrophenphthalat, 1-Methylimidazol, 2-Morpholinoethansulfonsäure-Monohydrat, 3-Morpho-linopropansulfonsäure, Natriumacetat-Trihydrat, Natriumcarbonat, tri-Natriumcitrat-Dihydrat, Natriumdihydrogenphosphat-Monohydrat, tretra-Natriumdiphosphat-Decahydrat, Natriumhydrogencarbonat, di-Natrium-hydrogenphosphat-Dihydrat, di-Natriumoxalat, di-Natriumtetraborat-Decahydrat, Piperazin-1,4-bis(2-ethansulfonsäure), Piperazin-1,4-bis-(2-ethansulfonsäure) Mononatriumsalz, Triethanolamin, Triethanolaminhydrochlorid, 2,4,6-Tri-methylpyridin, Tris(hydroxymethyl)-aminomethan, Tris(hydroxymethyl)-aminomethanhydrochlorid, N-[Tris(hydroxymethyl)-methyl]-2-amino-ethansulfon-säure und N-[Tris(hydroxymethyl)-methyl]-3-aminopropansulfonsäure bestehenden Gruppe ausgewählten Puffersubstanz e) erwiesen.

Die Art und Menge des eingesetzten Puffersalzes e) werden bei Formulierung des Dentalmaterials als Zweikomponentensystem vorzugsweise so gewählt, dass der pH-Wert der Basiskomponente A 5 bis 11, besonders bevorzugt 5 bis 9 und ganz besonders bevorzugt 5,5 bis 8,5 sowie der pH-Wert der Katalysatorkomponente B 6 bis 14, besonders bevorzugt 7 bis 13 und ganz besonders bevorzugt 11 bis 13 beträgt.

Bei Formulierung als Zweikomponentensystem enthält vorzugsweise die Basiskomponente A, bezogen auf die Komponente A, 0 bis 2 mmol/g, besonders bevorzugt 0,001 bis 1 mmol/g und ganz besonders bevorzugt 0,01 bis 0,2 mmol/g; sowie sowie die Katalysatorkomponente B, bezogen auf die Komponente B, 0 bis 2 mmol/g, besonders bevorzugt 0,001 bis 1 mmol/g und ganz besonders bevorzugt 0,01 bis 0,2 mmol/g wenigstens eines Puffersalzes e).

Als verstärkende Füllstoffe b₁) eignen sich hochdisperse, aktive Füllstoffe mit einer BET-Oberfläche von wenigstens 50 m²/g und/oder einer Primärpartikelgröße von kleiner gleich 100 nm, besonders bevorzugt kleiner gleich 80 nm. Besonders geeignet sind solche mit einer Primärpartikelgröße im Nanometerbereich, welche als Aggregate und/oder Agglomerate vorliegen können. Bevorzugt ist der wenigstens eine verstärkende Füllstoffe b₁) eine Substanz ausgewählt aus der Gruppe, welche aus Aluminiumhydroxid, Zinkoxid, Titandioxid, Zirkoniumoxid, Siliciumdioxid sowie gefällter und/oder pyrogener Kieselsäure besteht. Selbstverständlich können die zuvor genannten Verbindungen einzeln oder in beliebiger Kombination miteinander eingesetzt werden, und zwar auch sowohl in hydrophiler als auch in hydrophobierter Form.

Ferner bevorzugt liegt der wenigstens eine verstärkende Füllstoff b₁) in Form von Nanopartikeln, als faser- oder blättchenförmiger Füllstoff, bspw. mineralischer, faserförmiger Füllstoff, oder als synthetischer, faserförmiger Füllstoff vor.

In Weiterbildung des Erfindungsgedankens wird vorgeschlagen, bei Formulierung als Zweikomponentenmaterialien vorzugsweise in der Basiskomponente A, verstärkende Füllstoffe b₁) vorzusehen, die einen Wassergehalt von maximal 0,5 Gew.-%, besonders bevorzugt von maximal 0,3 Gew.-%, ganz besonders bevorzugt von maximal 0,15 Gew.-% aufweisen und höchst bevorzugt absolut bzw. im Wesentlichen wasserfrei sind, wobei der Wassergehalt über Karl-Fischer Titration bestimmt wird.

Gemäß einer weiteren besonderen Ausführungsform der vorliegenden Erfindung, weist der wenigstens eine verstärkende Füllstoff b₁) mit einer BET-Gemäß einer weiteren besonderen Ausführungsform der vorliegenden Erfindung, weist der wenigstens eine verstärkende Füllstoff b₁) mit einer BET-Oberfläche von größer 50 m²/g in der Basiskomponente A einen pH-Wert von 5 bis 11, bevorzugt von 5 bis 9 und besonders bevorzugt von 5,5 bis 8,5, auf. Auf diese Weise wird eine Degradierung der alkoxysilylfunktionellen Polyether während der Lagerung vermieden.

Bei Formulierung als Zweikomponentensystem enthält vorzugsweise die Basiskomponente A, bezogen auf die Komponente A, 0 bis 50 Gew.-%, besonders bevorzugt 0,1 bis 40Gew.-% und ganz besonders bevorzugt 0,1 bis 30 Gew.-%, und die Katalysatorkomponente B, bezogen auf die Komponente B, 0 bis 50 Gew.-%, besonders bevorzugt 0,1 bis 40 Gew.-% und ganz besonders bevorzugt 0,1 bis 30 Gew.-% wenigstens eines verstärkenden Füllstoffs b₁).

Als nichtverstärkende Füllstoffe b₂) eignen sich prinzipiell dieselben Substanzen wie für die verstärkende Füllstoffe b₁), wobei die nichtverstärkenden jedoch zwingend eine BET-Oberfläche von weniger als 50 m²/g (Schriftenreihe Pigmente Degussa Kieselsäuren, Nummer 12, Seite 5 sowie Nummer 13, Seite 3) aufweisen. Bevorzugt ist der wenigstens eine nichtverstärkende Füllstoffe b₂) eine Substanz ausgewählt aus der Gruppe, welche aus Erdalkalimetalloxiden, Erdalkalimetallhydroxiden, Erdalkalimetallfluorid, Erdalkalimetallcarbonaten, Calciumapatit (Ca₅[(F, Cl, OH, ½CO₃) | (PO₄)₃], insbesondere Calciumhydroxylapatit (Ca₅[(OH) | (PO₄)₃]), Titandioxid, Zirkoniumoxid, Aluminiumhydroxid, Siliciumdioxid, gefällter Kieselsäure und Calciumcarbonat besteht. Selbstverständlich können die zuvor genannten Verbindungen einzeln oder in beliebiger Kombination miteinander eingesetzt werden, und zwar auch sowohl in hydrophiler als auch in hydrophobierter Form.

Vorzugsweise weisen die eingesetzten nichtverstärkenden Füllstoffe b₂) eine mittlere Korngröße von größer als 0,1 µm (Ullmann Encyclopädie der Technischen Chemie, Band 21, Seite 523) auf.

In Weiterbildung des Erfindungsgedankens wird bei Formulierung des Dentalmaterials als Zweikomponentensystem vorgeschlagen, dass der wenigstens eine nichtverstärkende Füllstoff b₂) in der Basiskomponente A einen Wasserge-halt von maximal 0,5 Gew.-%, besonders bevorzugt maximal 0,1 Gew.-%, ganz besonders bevorzugt maximal 0,05 Gew.-% aufweist und höchst bevorzugt absolut bzw. im Wesentlichen wasserfrei ist.

Gemäß einer besonderen Ausführungsform der vorliegenden Erfindung weist der wenigstens eine nichtverstärkende Füllstoff b₂) in der Basiskomponente A einen pH-Wert von 5 bis 11, bevorzugt von 5 bis 9 und besonders bevorzugt von 5,5 bis 8,5, auf, um eine Degradierung der alkoxysilylfunktionellen Polyether während der Lagerung zu vermeiden.

Vorzugsweise enthält die Basiskomponente A des erfindungsgemäßen Dentalmaterials, bezogen auf die Komponente A, 0 bis 80 Gew.-%, besonders bevorzugt 0,05 bis 75 Gew.-% und ganz besonders bevorzugt 0,1 bis 70 Gew.-%, und die Katalysatorkomponente B, bezogen auf die Komponente B, 0 bis 80 Gew.-%, besonders bevorzugt 0,05 bis 75 Gew.-% und ganz besonders bevorzugt 0,1 bis 70 Gew.-% wenigstens eines nichtverstärkenden Füllstoffs b₂).

In Weiterbildung des Erfindungsgedankens wird vorgeschlagen, dass bei Formulierung als Zweikomponentenmaterial die in der Katalysatorkomponente B enthaltenen verstärkenden und/oder nichtverstärkenden Füllstoffe einen pH-Wert zwischen 6,0 und 11,0 und ganz besonders bevorzugt solche mit einem pH-Wert zwischen 7,0 und 10,0 aufweisen.

Insgesamt beträgt der Gesamtgehalt an Füllstoffen sowohl bei der Formulierung des Dentalmaterials als Ein- als auch als Zweikomponentensystem, bezogen auf die Gesamtmischung 0,001 bis 80 Gew.-%, bevorzugt 0,01 bis 80 Gew.-%, besonders bevorzugt 0,1 bis 75 Gew.-% und ganz besonders bevorzugt 0,2 bis 70 Gew.-%.

Als alkoxysilylfunktionelle Polyether a) können prinzipiell alle Polyether enthaltend Alkoxysilylgruppen eingesetzt werden, wobei das Polyetherrückgrat linear und/oder verzweigt und beispielsweise aus Polyethylenoxid, Polypropylenoxid, Polytetrahydrofuran und/oder deren Copolymeren aufgebaut sein kann, wobei diese Monomere statistisch, blockweise oder in einer taktischen Ordnung vorliegen können. Als Starter für die Polyether und/oder Copolymere können ein- oder mehrwertige Alkohole verwendet werden, wie bspw. Methanol, Butanol, Glycerin, Trimethylpropan, Pentaerythrit und Sorbitol. Beispielsweise können Copolymere aus Polytetrahydrofuran mit Polyethylenoxid oder aus Polyethylenoxid und Polypropylenoxid eingesetzt werden, wobei reines Polypropylenoxid besonders bevorzugt ist. Ferner bevorzugt sind Polyether mit seitenständigen Alkylgruppen, wobei jede oder wenigstens jede zehnte Monomerstruktureinheit eine seitenständige Alkylgruppe trägt. Geeignete Handelsprodukte sind Acclaim 4200, Acclaim 6320N, Acclaim 12200, Acclaim 8200 und Acclaim 6300 der Bayer AG, Polyglycol P41/300 und Polyglycol P41/3000 (Clariant) sowie Poly-(ethylenglycol-ran-propylenglycol) (Aldrich). Bevorzugt weisen die Polyether a) ein zahlengemitteltes Molekulargewicht von 500 bis 25.000 g/mol und besonders bevorzugt von 5.000 bis 20.000 g/mol auf.

Ein Auswahlkriterium für den erfindungsgemäß geeigneten Polyether ist neben der Kettenlänge (Elastizität), der Alkoxysilylfunktionalisierung (Aushärte-Kinetik) und der Anzahl der Urethan-/Harnstoffgruppen (Viskosität, Rheologie) die Hydrophilie des Polyethers, die über die Anzahl, Struktur und Polarität der Monomerwiederholungseinheiten des Polyetherpolymers bestimmt wird. Die Hydrophilie für ein Dentalabformmaterial muss einerseits ausreichend hoch sein, um ein gutes Anfließen an feuchte Zahnsubstanz sicherzustellen (niedrige Kontaktwinkel), aber andererseits darf das Material nicht zu hydrophil sein, da sonst Wasser, Feuchtigkeit oder Speichel während der Abdrucknahme bzw. beim Desinfizieren bzw. beim Ausgießen mit Gips zur Quellung des Dentalmaterials führt und damit die erforderliche Dimensionsgenauigkeit nicht mehr gegeben ist. Die Hydrophilie des Polyethers ist darüber hinaus auch unter anderem für die Löslichkeit des erfindungsgemäßen Katalysators mit verantwortlich.

Gemäß einer besonderen Ausführungsform der vorliegenden Erfindung weist der wenigstens eine alkoxysilylfunktionelle Polyether einen Gehalt an Polyethergruppen zwischen 5 und 30 mmol/g und besonders bevorzugt zwischen 10 und 25 mmol/g auf.

Vorzugsweise ist die Alkoxysilylstruktureinheit bzw. sind die Alkoxysilylstruktureinheiten des Polyethers a), bezogen auf das Polymerrückgrat, terminal angeordnet und fallen unter die allgemeine Formel IV

- SiR⁵R⁶R⁷

worin R⁵, R⁶ und R⁷ unabhängig voneinander Alkoxy-, Alkyl-, Aryl-, Aralkyl-, Alkylarylgruppen oder Wasserstoff, bevorzugt Butoxy-, Propoxy-, Ethoxy-, Methoxy-, Hexyl-, Pentyl-, Butyl-, Propyl-, Ethyl- oder Methylgruppen, besonders bevorzugt Ethoxy-, Methoxy-, Ethyl- oder Methylgruppen, sind mit der Maßgabe, dass mindestens einer der vorgenannten Reste, vorzugsweise zwei oder alle drei Reste, Alkoxygruppen sind.

In Weiterbildung des Erfindungsgedankens wird vorgeschlagen, dass der wenigstens eine Polyether a) einen Alkoxygruppengehalt von 0,02 bis 12 mmol/g, besonders bevorzugt von 0,04 bis 6 und ganz besonders bevorzugt von 0,04 bis 3 mmol/g, aufweist.

Durch die Art und Anzahl der Alkoxygruppen pro Siliziumatom lässt sich die Kondensationskinetik und damit die Verarbeitungs- und Abbindezeit des Dentalmaterials einstellen. Bevorzugt werden diese Parameter derart gewählt, dass die Verarbeitungszeit 30 Sekunden bis 3 Minuten, besonders bevorzugt zwischen 1 und 2,5 Minuten und ganz besonders bevorzugt zwischen 1,5 und 2 Minuten und/oder die nach ISO 4823 (Ausgabe 1992) bestimmte Abbindezeit im Patientenmund (sog. Mundverweildauer) maximal 15 Minuten, besonders bevorzugt maximal 10 Minuten und ganz besonders bevorzugt maximal 7 Minuten und höchst bevorzugt 6 Minuten beträgt.

Vorzugsweise weist der wenigstens eine Polyether a) als dritte Struktureinheit (neben den terminalen Alkoxygruppen und den Polyethergruppen) jeweils an den terminalen Alkoxysilylgruppen angeordnete Alkylenspacer, welche bevorzugt C₁-C₆-Alkylgruppen, besonders bevorzugt C₁-C₃-Alkylgruppen, ganz besonders bevorzugt Ethylengruppen und/oder Methylengruppen und höchst bevorzugt Methylengruppen sind, auf.

Zudem kann der wenigstens eine Polyether a) synthesebedingt als vierte Struktureinheit 0 bis 8 mmol/g, besonders bevorzugt 0 bis 4 mmol/g und ganz besonders bevorzugt 0,02 bis 2 mmol/g insbesondere 0,1 bis 0,4 mmol/g Urethangruppen und/oder 0 bis 8 mmol/g und besonders bevorzugt 0,02 bis 2 mmol/g insbesondere 0,1 bis 0,4 mmol/g Harnstoffgruppen aufweisen. Insgesamt sollte der Gehalt an Urethan- bzw. Harnstoffgruppen pro Molekül so gering wie möglich gehalten werden, um die intermolekularen Wechselwirkungen zwischen den einzelnen Polyetherketten zu minimieren, um die durch den Polyetherzusatz begründete Viskosität so gering wie möglich zu halten, was den Zusatz höherer Mengen an Füllstoffen in dem Dentalmaterial ermöglicht und somit mehr Formu-lierungsfreiräume und kostengünstigere Rezepturen einräumt.

Gemäß einer weiteren besonderen Ausführungsform der vorliegenden Erfindung sind daher solche Alkoxysilylpolyether-Polymere bevorzugt, die innerhalb der Polyetherkette keine Urethan- oder Harnstoffgruppen enthalten und die an jedem Kettenende höchstens eine bzw. nicht mehr als eine Urethan- oder Harnstoffgruppe und höchstens eine bzw. nicht mehr als eine (Alkoxy)silylgruppe und höchstens eine bzw. nicht mehr als eine Methylenspacergruppe tragen (sog. α-aktivierter Alkoxysilylpolyether). Mit den gemäß dieser Ausführungsform der vorliegenden Erfindung eingesetzten Alkoxysilylpolyethern mit hoher Molmasse und nur einer Urethangruppen an jedem Kettenende wird eine im Vergleich zu den nach dem Stand der Technik eingesetzten Polyurethan-Alkoxysilylpolyethern niedrige Viskosität erzielt werden, wodurch mehr Füllstoffe eingesetzt werden können, was zu niedrigen Herstellkosten führt. Im Unterschied dazu sind die in einem Teil des Standes der Technik vorgesehen alkoxysilylfunktionelle Polyurethane für den Einsatz als Dentalmaterial weniger bzw. nicht geeignet, da aufgrund des hohen Urethangehalts im Polymer durch intermolekulare Wasserstoffbrückenbindungen zwischen den Urethangruppen der einzelnen Polymere hohe Viskositäten resultieren. Dies hat den Nachteil, dass Formulierungsfreiheit verloren geht und relativ teure Rezepturen entstehen, da nur ein relativ geringer Füllstoffanteil in die Polymere eingearbeitet werden kann, da sonst eine zu hohe Verarbeitungskonsistenz entstehen würde. Weiterhin sind bei den Polyurethanen im Vergleich zu Polyethern bei gleicher Viskosität nur kürzere Polymerketten zu realisieren. Kurze Polymerketten stehen guten elastischen Eigenschaften, wie sie bei Zahnabformmaterialien erforderlich sind, entgegen.

Durch den Einsatz der vorgenannten α-aktivierten Alkoxysilylpolyether werden hydrophile, lagerstabile Zweikomponenten-Dentalabformmassen erhalten, welche mit einer erfindungsgemäß als Katalysator einzusetzenden organischen Base überraschend schnell durch Kondensationsreaktion vernetzen.

Gemäß der vorliegenden Erfindung sind die einzelnen Struktureinheiten des wenigstens einen Polyethers a) folgendermaßen angeordnet: 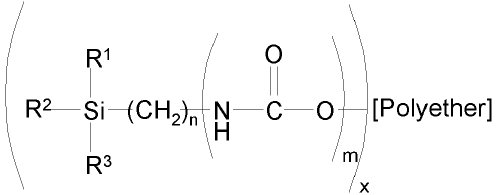 worin R¹, R² und R³ unabhängig voneinander Alkoxy-, Alkyl-, Aryl-, Aralkyl-, Alkylarylgruppen oder Wasserstoff, bevorzugt Butoxy-, Propoxy-, Ethoxy-, Methoxy-, Hexyl-, Pentyl-, Butyl-, Propyl-, Ethyl- oder Methylgruppen, besonders bevorzugt Ethoxy-, Methoxy-, Ethyl- oder Methylgruppen, sind mit der Maßgabe, dass mindestens einer der vorgenannten Reste, vorzugsweise zwei oder alle drei Reste, Alkoxygruppen sind, sowie
x=1 bis 6, bevorzugt x=2 bis 4 und ganz besonders bevorzugt x=2, n=1 bis 6, bevorzugt n=1 bis 3 und ganz besonders bevorzugt n=1 sowie m=0 oder 1, besonders bevorzugt m=1, ist
oder 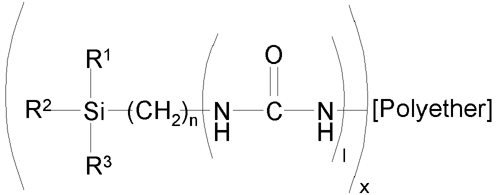 worin R¹, R² und R³ unabhängig voneinander Alkoxy-, Alkyl-, Aryl-, Aralkyl-, Alkylarylgruppen oder Wasserstoff, bevorzugt Butoxy-, Propoxy-, Ethoxy-, Methoxy-, Hexyl-, Pentyl-, Butyl-, Propyl-, Ethyl- oder Methylgruppen, besonders bevorzugt Ethoxy-, Methoxy-, Ethyl- oder Methylgruppen, sind mit der Maßgabe, dass mindestens einer der vorgenannten Reste, vorzugsweise zwei oder alle drei Reste, Alkoxygruppen sind, sowie
x=1 bis 6, bevorzugt x=2 bis 4 und ganz besonders bevorzugt x=2, n=1 bis 6, bevorzugt n=1 bis 3 und ganz besonders bevorzugt n=1 sowie l=0 oder 1, besonders bevorzugt l=1 ist.

Gemäß einer weiteren besonderen Ausführung der vorliegenden Erfindung ist der Alkylspacer in den vorgenannten Struktureinheiten Methylen (n=1).

Die Herstellung dieser alkoxysilylfunktionellen Polyether ist bekannt und wird beispielsweise in der DE 101 04 079 A1, EP 0 629 819 B1, DE 101 39 132, US 4,906,707, EP 0 372 561 A1, EP 1 303 560 A1 und EP 0 170 865 B1, welche hiermit als Referenz eingeführt und als Teil der Offenbarung gelten, beschrieben. Beispiele für kommerziell erhältliche und im Rahmen der vorliegenden Erfindung geeignete Polyether sind MS Polymer S 203H, MS Polymer S 303H (Kaneka), Polymer XP ST55, ST50, ST51, ST53 (Hanse), SLM 414000 (Wacker), SLM 414001 (Wacker), Baycoll XP 2458 und Desmoseal XP 2447 (Bayer AG), wobei der unter dem Handelsnamen SLM 414000 vertriebene Dimethoxy(methyl)silylmethylcarbamat-terminierte Polyether: 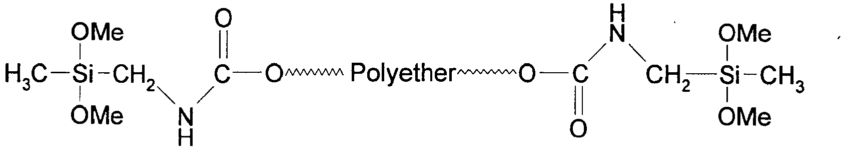 und Dimethoxy(methyl)silylmethylharnstoff-terminierte Polyether: 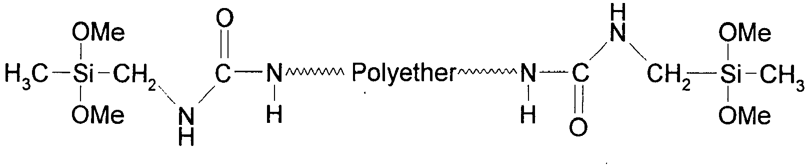 besonders bevorzugt sind.

In Weiterbildung des Erfindungsgedankens wird vorgeschlagen, dem erfindungsgemäßen Dentalmaterial ein oder mehrere der folgenden Zusatz- bzw. Hilfsstoffe zuzusetzen:
f) Thixotropierungsmittel,
g) Wasserfänger,
h) Pastenbildner,
i) Tensid,
j) Wirkstoff,
k) Weichmacher,
l) optische Abtastung ermöglichende Substanz,
m) Geschmacks- und/oder Geruchsstoff,
n) Diagnostik ermöglichende Substanz,
o) Fluoridisierungsmittel,
p) Bleichsubstanz,
q) Desensibilisierungsmittel,
r) Haftverbundvermittler,
s) Farbstoff,
t) Indikator,
u) Stabilisator (Antioxidanz, Radikalfänger).

Dem erfindungsgemäßen Dentalmaterial können optional Thixotropierungsmittel f) zugesetzt werden, wobei sich zu diesem Zweck insbesondere hochmolekulare Polyether, wie Polyethylenglykol, Polypropylenglycol, Polyethylenglykol-Polypropylenglykol-Copolymere, Poly-Tetrahydrofuran, Kohlenwasserstoffwachse, Amidwachse, Triglyceride, Kieselsäuren und Silicate als besonders geeignet erwiesen haben.

Gemäß einer weiteren besonderen Ausführungsform der vorliegenden Erfindung weisen die Dentalmaterialien, wenn als Zweikomponentensystem formuliert vorzugsweise in der Basiskomponente A, wenigstens einen Wasserfänger g) auf, welcher besonders bevorzugt aus der Gruppe, welche aus Alkoxysilanen, Titanaten, wie Tetraisopropyltitanat, Zirkonaten, wie Tetrapropylzirkonat, Zeolithen, Aluminiumsulfat, wasserfreiem Calciumsulfat (bspw. Drierite®), Blaugel und/oder Oxazolidinen besteht, ausgewählt ist.

In Weiterbildung des Erfindungsgedankens wird vorgeschlagen, als Wasserfänger g) ein oder mehrere funktionelle Alkoxysilane einzusetzen, da durch solche Verbindungen zusätzlich die Vernetzungsgeschwindigkeit, die Struktur und die Eigenschaften des resultierenden Elastomers eingestellt werden können. Bevorzugt ist das wenigstens eine funktionelle Alkoxysilan eine Verbindung der allgemeinen Formel V

R⁸₄₋ₓ-Si-R⁹ₓ

mit R⁸=H, Alkyl, Alkenyl, -(CH₂)ₙ-Z, wobei n=1 bis 6,
R⁹=Alkoxy,
Z=NH₂, NHR, NR₂, wobei R=Alkyl, Aminoalkyl, -C(O)OCH₃, sowie
x=1, 2, 3 oder 4,
wobei besonders bevorzugt R⁸= Alkenyl, insbesondere Vinyl, oder -(CH₂)ₙ-Z mit Z=NHR und n=1 oder 3, insbesondere n=1, und/oder
x=3 und/oder R= -C(O)OCH₃.

Besonders bevorzugt ist das wenigstens eine funktionelle Alkoxysilan g) Vinyltrimethoxysilan und/oder N-Trimethoxysilylmethyl-O-methylcarbamat und/oder eine Verbindung der folgenden Formel: 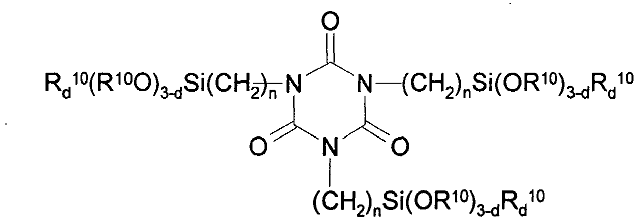
worin n = 1 bis 6, bevorzugt n = 1 oder 3, besonders bevorzugt n = 1,
d = 0 oder 1, und
R¹⁰ = ein linearer oder verzweigter C₁-C₃₀-Alkylrest, in dem die Wasserstoffatome teilweise durch Halogenatome, OH-, NH₂-, NO₂- oder auch weitere C₁-C₆-Alkylreste substituiert sein können, sind.

Die zuvor genannten Verbindungen sind reaktive Silane, die als Wasserfänger zur Beseitigung von in der Komponente A der Dentalzusammensetzung noch vorhandenen Wasserspuren fungieren.

Des weiteren enthalten die erfindungsgemäßen Zweikomponenten-Dentalmaterialien vorzugsweise, und zwar wenn als Zweikomponentensystem formuliert besonders bevorzugt in der Katalysatorkomponente B, wenigstens einen Pastenbildner h), da dieser die Einstellung einer pastenartigen Konsistenz, bspw. dünn-, mittel- oder zähfließend, sowie eine homogene Vermischung der erfindungsgemäß eingesetzten organischen Base mit Wasser und der verstärkenden und nichtverstärkenden Füllstoffe ermöglicht. Vorzugsweise wird als wenigstens ein Pastenbildner h) eine Verbindung ausgewählt aus der Gruppe, welche aus Polyethern, Polyvinylpyrrolidonen, Polyurethanen, Polyestern, Wachsen, Vaseline, Paraffinölen, Siliconölen, mehrwertigen Alkoholen, Propylenglycolen, Polypropylenglycolen, Ethylenglycolen, Polyethylenglycolen, Copolymerisaten aus N-Vinylpyrrolidon und Vinylacetat, Cärboxymethyl-, Methyl-, Hydroxyethyl-, Hydroxypropyl-Cellulose, Polysacchariden und Poly(meth)acrylsäuren besteht, eingesetzt. Selbstverständlich können die erfindungsgemäßen Dentalmaterialien auch eine beliebige Kombination zweier oder mehrerer der zuvor genannten Verbindungen enthalten.

Besonders bevorzugt sind hydrophile Pastenbildner, in denen sich die erfindungsgemäße Katalysatorbase mit Wasser homogen mischen lässt. Die Mischbarkeit kann durch Zusatz von Tensiden noch verbessert werden. Besonders bevorzugte Vertreter sind Polyether, Polyurethane, Polyester, mehrwertige Alkohole, insbesondere Propylenglycole, Polypropylenglycole, Ethylenglycole, Polyethylenglycole, Butylenglycole, Polybutylenglycole, Glycerin sowie Mischungen und Copolymere hiervon.

Bei den ggf. als Tensid, Emulgator und/oder Stabilisator eingesetzten Verbindungen i) handelt es sich vorzugsweise um anionische Tenside, besonders bevorzugt um Alkylsulfate, Alkylbenzolsulfonate oder Alkylbenzolphosphate, kationische Tenside, besonders bevorzugt Tetraalkylammoniumhalogenide, nichtionische Tenside, besonders bevorzugt Alkyl- und Alkylphenyl-Polyalkylalkylenoxide, Fettsäurealkoxylate, Fettalkoholalkyloxylate sowie deren Alkylether und Alkylester, Fettsäurealkylolamide, Saccharosefettsäureester, Trialkylaminoxide, Silicontenside (z.B. Silwet L77, Tegopren 5878) oder Fluortenside, oder um amphotere Tenside, besonders bevorzugt sulfatierte oder oxyethylierte Kondensationsprodukte aus Alkenylphenolen und Formaldehyd, Ethylenoxid-propylenoxid-Blockpolymerisate oder modifizierte Polysiloxane. Mit Vorteil können auch in den alkoxysilylfunktionellen Polyether a) einpolymerisierbare Tenside, wie sie in der US 4,160,778, die hiermit als Referenz eingeführt und als Teil der Offenbarung gilt, offenbart sind, eingesetzt werden. Zusätzlich oder alternativ dazu können auch Derivate der zuvor genannten Tenside eingesetzt werden, bspw. solche, die über funktionelle Gruppen, wie -OH, -CH=CH₂, -OCO-(CH₃)C=CH₂ sowie Alkoxysilylgruppen, verfügen. Zudem können, wenn auch weniger bevorzugt, andere, dem Fachmann bekannte Tenside eingesetzt werden. Mit Silicontensiden konnten überraschenderweise in der Polyethermatrix sehr niedrige Kontaktwinkel erzielt werden (Methode: liegender Tropfen).

Diese Mischungen zeichnen sich durch eine ausgezeichnete Benetzbarkeit und ein hervorragendes Anfließverhalten an feuchte Zahn- und Gewebssubstanz aus. Trotz dieser guten hydrophilen Eigenschaften quillt das Material nicht bei Kontakt mit wässrigen Medien, wie Wasser, Speichel, Blut, Desinfektionsbad oder wässrigem Gipsbrei. Die gute initiale Benetzbarkeit der Mischungen ist wichtig für die detailgetreue Abformung des Abformmaterials in dem Patientenmund während der Verarbeitung und dem ersten Kontakt mit feuchter Mund-/Zahnsubstanz und drückt sich durch einen niedrigen Kontaktwinkel von kleiner 50°, insbesondere kleiner gleich 45°, gemessen mit einem Kontaktwinkelmessgerät der Firma Krüss bei 20 °C mit der Messmethode "liegender Tropfen", aus. Zudem zeichnet sich auch das ausgehärtete Abformmaterial zum Zeitpunkt des Ausgießens mit Gips (direkt oder 2 Stunden nach dem Aushärten) durch einen Kontaktwinkel von kleiner 60°, insbesondere kleiner gleich 55°, aus.

Zudem können die erfindungsgemäßen Dentalmaterialien eine oder mehrere Wirkstoffe j) enthalten, welche in Abhängigkeit von deren chemischen Funktionalität bei Formulierung als Zweikomponentensystem in der Basiskomponente A oder der Katalysatorkomponente B enthalten sein können. Zu den erfindungsgemäß einzusetzenden Wirkstoffen zählen insbesondere Adstringentien, wie Epinephrine, antibakteriell und/oder antifugal wirkende-Substanzen, wie Hexitidine (bspw. 5-Amino-1, 3-bis(2-ethylhexyl)-5-methylhexahydropyrimidin), Triclosane (bspw. 2,4,4'-Trichloro-2-hydroxydiphenylether) und Chlorhexidin: 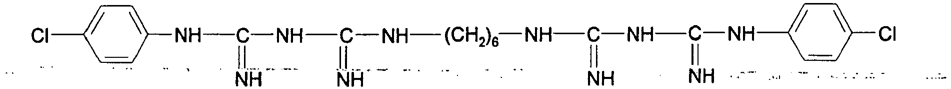 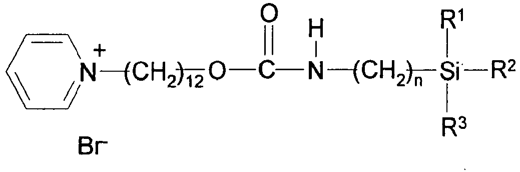 worin R¹, R² und R³ unabhängig voneinander Alkoxy-, Alkyl-, Aryl-, Aralkyl-, Alkylarylgruppen oder Wasserstoff, bevorzugt Butoxy-, Propoxy-, Ethoxy-, Methoxy-, Hexyl-, Pentyl-, Butyl-, Propyl-, Ethyl- oder Methylgruppen, besonders bevorzugt Ethoxy-, Methoxy-, Ethyl- oder Methylgruppen, sind mit der Maßgabe, dass mindestens einer der vorgenannten Reste, vorzugsweise zwei oder alle drei Reste, Alkoxygruppen sind.

Als Weichmacher k) kommen insbesondere nicht reaktive Polyether, Polyester, Polyurethane, Phthalate, Mono-, Di-, Tri- oder höherwertige Ester, insbesondere Acetyltributylcitrat, Mesamoll ® (Bayer) und Triglyceride in Betracht, welche bei Formulierung als Zweikomponentensystem je nach deren chemischen Natur der Komponente A und/oder der Komponente B zugesetzt werden.

Als die optische Lesbarkeit/Abtastung ermöglichenden Verbindungen l) können alle dem Fachmann zu diesem Zweck bekannte Substanzen, insbesondere Metallpulver, Metallpigmente, Metallicpigmente, Zinkoxid, Zirkonoxid und Titandioxid, eingesetzt werden, welche bei Formulierung als Zweikomponentensystem je nach deren chemischen Natur der Komponente A und/oder der Komponente B zugesetzt werden.

Zudem können die erfindungsgemäßen Dentalmaterialien in einer der beiden oder in beiden Komponenten übliche Geschmacks- und/oder Geruchsstoffe m) und/oder für die Diagnostik nützliche Zusätze n) enthalten, wie sie bspw. in der EP 1 339 373, PCT/EP00/05418 und DE 100 61 195 beschrieben sind.

Als Fluoridierungshilfsstoffe o) haben sich insbesondere Natriumfluorid, Kaliumfluorid, Ammoniumfluorid, Fluorphosphate und Aminfluoride, wie N'-Octadecylbimethylendiamin-N, N, N'-bis(2-ethanol)-dihydrofluorid (wie in ZM 93, Nummer 15, Seite 32 ff. beschrieben), als geeignet erwiesen, welche bei Formulierung als Zweikomponentensystem ebenfalls in Abhängigkeit von deren chemischen Natur der Komponente A und/oder der Komponente B zugesetzt sein können.

Zudem kann das erfindungsgemäße Dentalmaterial, bei Formulierung als Zweikomponentensystem in der Komponente A und/oder der Komponente B, als Bleichsubstanz p) eine oder mehrere verschiedene Peroxide enthalten, welche vorzugsweise aus der Gruppe, welche aus Alkalimetall- und Erdalkalimetallperoxiden, Wasserstoffperoxid sowie Carbamidperoxid besteht, ausgewählt sind.

Beispiele für geeignete Desensibilisierungsmittel q) sind Kaliumsalze, wie Kaliumnitrat, Nelkenöl und Eugenol.

Als Haftverbundvermittler r), bspw. zur Ausbildung eines Haftverbundes zwischen dem Abformmaterial und einem Abformlöffel aus Edelstahl und/oder Kunststoff, eignen sich insbesondere Alkoxysilane, Epoxysilane, Aminosilane und Methacrylatsilane.

Beispiele für geeignete Farbstoffe s) sind Farbstoffpigmente in Form von Al-, Ca-, Ba-Oxiden/verlackter Farbstoff, welche wie die zuvor beschriebenen Hilfsstoffe, sofern nicht anders angegeben, bei Formulierung als Zweikomponentensystem in Abhängigkeit von deren chemischen Natur der Komponente A und/oder der Komponente B zugesetzt sein können.

Des weiteren können dem erfindungsgemäßen Dentalmaterial bei Formulierung als Zweikomponentensystem in der Komponente A und/oder der Komponente B Farbstoffindikatoren t) zugesetzt sein, welche ihre Farbe in Abhängigkeit von dem pH-Wert, bspw. aufgrund von pH-Wert-Änderungen beim Vermischen der Komponenten A und B, oder beim Kontakt mit Wasser ändern.

Als Stabilisatoren und/oder Antioxidantien u) können den erfindungsgemäßen Zweikomponenten-Dentalmaterialien insbesondere aus der aus polymerem Trimethyl-dihydrochinolin, Diphenylderivaten, Phenothiazin, Phenyl-α-naphthylamin, 4, 4'-Methylen-bis-2,6-di-tert.-butylphenol, Butylhydroxytoluol, Butylhydroxyanisol (BHA) und Methoxyphenol (Hydroxyanisol) bestehenden Gruppe ausgewählte Verbindungen eingesetzt werden. Beispiele für derartige Verbindungen sind die von der Firma Ciba-Geigy kommerziell erhältlichen Produkte Irganox 1010, 1076, 1035, MD 1024, Irgafos 168, 38, Irgacor 252 LD/252FC, 1405, 1930, 153, Tinuvin 328, P, 384, 900, 928, 327, 1130, 400, 292, 144, 123, 622 sowie Chimassorb 119.

Vorzugsweise wird das erfindungsgemäße Zweikomponenten-Dentalmaterial in geeigneten Primärverpackungen, wie Tuben, Dosen, und besonders bevorzugt in Kartuschen und Schlauchbeuteln, wie sie bspw. in der EP 0 723 807 A2, EP-A-0 541 972, PCT/EP/980193, EP-A-0 492 412, EP-A-0 492 413 und EP 0 956 908 A1, welche hiermit als Referenz eingeführt und somit als Teil der Offenbarung gelten, beschrieben sind, gelagert und auf die spätere Verwendung zugeschnitten proportioniert ist.

Ein weiterer Gegenstand der vorliegenden Erfindung sind Mischungen, welche durch Vermischen der Komponenten A und B des zuvor beschriebenen erfindungsgemäßen Zweikomponenten-Dentalmaterials erhältlich sind. Vorzugsweise wird die Basiskomponente A mit der Katalysatorkomponente B in einem Verhältnis von 1:1 bis 20:1, besonders bevorzugt von 1:1 bis 10:1 und ganz besonders bevorzugt von 1:1, 2:1, 4:1 und 5:1, vermischt.

Im Folgenden wird die Erfindung anhand von den Erfindungsgedanken demonstrierenden, diesen aber nicht einschränkenden Beispielen erläutert.

### Beispiele 1 bis 18 (erfindungsgemäß)

### (Herstellung verschiedener Katalysatorkomponenten B mit unterschiedlichen erfindungsgemäßen freien organischen Basen mit einem in Acetonitril gemessenen pk_{BH+}-Wert von mindestens 20)

### Herstellung verschiedener Katalysatorkomponenten B

Verschiedene erfindungsgemäß einzusetzende organische Basen mit einem in Acetonitril gemessenen pk_{BH+}-Wert von mindestens 20 und gegebenenfalls einem Puffersalz als Zusatzstoff wurden in den in der Tabelle 1 angegebenen Mengen in 2,16 Teilen entmineralisiertem Wasser (Ampuwa, pH 5,8) gelöst. Anschließend wurde die Lösung in einem Vakuummischbecher mit 44 Teilen Polypropylenglycol mit einer Molmasse von 4000 g/mol, 45 Teilen Calciumcarbonat mit einer mittleren Korngröße von 6 µm und einer BET-Oberfläche von 1 m²/g und 5 Teile Calciumcarbonat mit einer mittleren Korngröße von 0,5 µm und einer BET-Oberfläche von 30 m²/g für 5 Minuten gemischt. Danach wurde weitere 30 Minuten unter Vakuum homogen gemischt.

Es wurden mittelfließende Materialien (ISO 4823) erhalten, die verschiedene Katalysatorkomponenten B des erfindungsgemäßen Abformmaterials auf Basis von Alkoxysilylpolyethern darstellen. Die Materialien wurden in Schlauchbeutel (Verbundfolie PE/AI/PE) abgefüllt und aufbewahrt.

### Herstellung einer Basiskomponente A

In einem Vakummischer wurden unter trockener Argon-Schutzgasatmosphäre 42 Teile eines Polypropylenglycols, das endständig über Urethangruppen und Methylenspacer mit Dimethoxymethylsilylgruppen funktionalisiert war, wobei das funktionalisierte Polypropylenglycol eine Viskosität bei 20°C von 10.000 mPa·s aufwies mit 53 Teilen eines getrockneten mit Trimethylsilylgruppen oberflächenmodifizierten Cristobalitfüllstoffs mit einer mittleren Korngröße von 7 µm, zwei Teilen einer getrockneten, hochdispersen, pyrogen hergestellten, hydrophobierten Kieselsäure mit einer BET-Oberfläche von 170 m²/g, einem Teil Vinyltrimethoxysilan und einem Teil N-Trimethoxysilylmethyl-O-methylcarbamat für 5 Minuten gemischt. Danach wurde weitere 30 Minuten unter Vakuum homogen gemischt.

Es wurde ein mittelfließendes Material (ISO 4823) erhalten, das die Basiskomponente A des erfindungsgemäßen Abformmaterials auf Basis von Alkoxysilylpolyethern darstellt. Das Material wurde in Schlauchbeutel (Verbundfolie PE/AI/PE) abgefüllt und aufbewahrt.

### Mischung der Katalysatorkomponenten B und der Basiskomponente A

Jeweils 1 Teil der zuvor beschriebenen Katalysatorkomponente B und 5 Teile der gemäß obiger Vorschrift hergestellten Basiskomponente A wurden mit Hilfe eines elektrischen Austraggeräts aus Schlauchbeuteln (Plug & Press System, Kettenbach GmbH & Co. KG) über einen dynamischen Mischer (Kettenbach GmbH & Co. KG) homogen gemischt.

Die Abbindezeiten der so hergestellten Dentalmaterialien auf Basis von Alkoxysilylpolyethern sind in der Tabelle 1 sowie weitere anwendungstechnische Eigenschaften, u.a. Verarbeitungszeit, Abbindezeit und Abbindezeit nach einem Thermostresstest von einer Woche bei 60°C, für das in Beispiel 3 erhaltene Dentalmaterial in den Tabellen 2, 3 und 4 zusammengefasst.

Die Beispiele 1 bis 18 zeigen, dass die erfindungsgemäßen Zusammensetzungen enthaltend als Katalysator eine starke Base mit einem in Acetonitril gemessenen pK_{BH+}-Wert von mehr als 20 überraschenderweise eine für kondensationsvernetzende Alkoxysilylpolyether-Systeme hervorragende Aushärtekinetik aufweisen. Es lassen sich schnellabbindende Abformmaterialien mit praxisgerechten Verarbeitungszeiten herstellen, wobei die ausgehärteten Materialien keine unerwünschte Klebrigkeit aufweisen, so dass diese als dentales Abformmaterial hervorragend geeignet sind. Der Fachmann erkennt, dass die Abbindezeit der einzelnen Beispiele durch Erhöhung bzw. Reduzierung der Menge an eingesetzter Katalysatorbase einfach auf einen gewünschten Wert eingestellt werden kann.

Wie insbesondere aus der Tabelle 4 für die in Beispiel 3 erhaltene Masse hervorgeht, erfüllen die erfindungsgemäßen Dentalmaterialien alle Anforderungen für ein funktionsfähiges dentales Abformmaterial, vor allem im Hinblick auf Shore A-Härten, Rückstellung nach der Verformung, Konsistenzen der Einzelkomponenten und der Mischung, lineare Maßänderungen und Kontaktwinkel. Insbesondere werden für die durch Kontaktwinkelmessung bestimmte Hydrophilie hervorragende Werte erhalten.

### Vergleichsbeispiele 1 bis3(nicht erfindungsgemäß)

Verschiedene organische Basen mit einem in Acetonitril gemessenen pk_{BH+}-Wert von weniger als 20 wurden in 5,0 Teilen entmineralisiertem Wasser (Ampuwa, pH 5,8) gelöst und gleichermaßen wie in den Beispielen 1 bis 18 zu einer Katalysatorkomponente B verarbeitet.

Jeweils 1 Teil der zuvor beschriebenen Katalysatorkomponenten B und 5 Teile der Basiskomponente A gemäß Beispiel 1 wurden mit Hilfe eines elektrischen Austraggeräts aus Schlauchbeuteln (Plug & Press System, Kettenbach GmbH & Co. KG) über einen dynamischen Mischer (Kettenbach GmbH & Co. KG) homogen gemischt.

Die Abbindezeiten der so hergestellten Materialien auf Basis von Alkoxysilylpolyethem sind in der Tabelle 1 zusammengefasst.

Wie aus der Tabelle 1 ersichtlich härten die in den Vergleichsbeispielen 1 bis 3 hergestellten Materialien im Unterschied zu den erfindungsgemäßen Formulierungen nicht aus.

### Vergleichsbeispiel 4 (nicht erfindungsgemäß)

### (Säurekatalysiertes kondensationsvernetzendes Dentalmaterial auf Basis von Alkoxysilylpolyethern gemäß Beispielen 3 und 5 der EP 1 226 808 A2)

Ein säurekatalysiertes Dentalmaterial nach dem Stand der Technik auf Basis von Alkoxysilypolyethern bestehend aus einer sauren Katalysatorkomponente B und einer Basiskomponente A wird gemäß Beispielen 3 und 5 der EP 1 226 808 A2 hergestellt und gemischt.

Die Verarbeitungszeit, Abbindezeit und die Abbindezeit nach einem Thermostress-test von einer Woche bei 60°C des gemäß Vergleichsbeispiel 4 hergestellten Dentalmaterials auf Basis von Alkoxysilylpolyethern ist in der Tabelle 2 zusammengefasst.

Im Vergleich zu den erfindungsgemäßen Dentalmaterialien auf Basis von Alkoxysilylpolyethern (Beispiel 3) zeigt das säurekatalysierte System gemäß der Beispiele 3 und 5 der EP 1 226 808 A2 nach Lagerung im Thermostresstest (eine Woche 60 °C) keine Aushärtung mehr. Dies zeigt, dass die Katalysatorkomponente aus Beispiel 3 der EP 122 6088 A2 instabil ist. Dies führt zu einer Verlängerung der Abbindezeit. Eine unabhängig von der Lagerzeit gleich bleibende Abbindezeit ist allerdings eine der wichtigsten Anforderungen an ein dentales Abformmaterial.

### Beispiele 3a bis 3e (erfindungsgemäß)

Der in Beispiel 3 hergestellten Katalysatorkomponente B wurden verschiedene adstringierende bzw. antibakterielle Zusätze in den in der Tabelle 3 wiedergegebenen Mengen zugegeben und wie in Beispiel 3 beschrieben in Schlauchbeutel (Verbundfolie PE/AI/PE) abgefüllt und aufbewahrt. Jeweils 1 Teil dieser Katalysatorkomponenten wurde mit 5 Teilen der gemäß Beispiel 3 hergestellten Basiskomponente A mit Hilfe eines elektrischen Austraggeräts aus Schlauchbeuteln (Plug & Press System, Kettenbach GmbH & Co. KG) über einen dynamischen Mischer (Kettenbach GmbH & Co. KG) homogen gemischt.

Die Abbindezeiten und Abbindezeit nach einem Thermostresstest von einer Woche bei 60°C der so hergestellten Dentalmaterialien sind in der Tabelle 3 zusammengefasst.

Wie aus der Tabelle 3 hervorgeht hat der Zusatz an adstringierenden bzw. antibakteriellen Zusätze keinen bzw. lediglich einen vernachlässigbar geringen Einfluss auf die Abbindezeit und die Lagerstabilität der erfindungsgemäßen Zusammensetzungen.

**Tabelle 1**

| **Zusammensetzung und Abbindezeit der Massen gemäß den Beispielen 1 bis 18 und den Vergleichsbeispielen 1 bis 3** | | | | | | | |
|---|---|---|---|---|---|---|---|
| **erfindungsgemäße Beispiele/ Vergleichsbeispiele** | **organische Base** | **Puffersalz** | **Base Anteile [%]** | **Puffersalz Anteile [%]** | **pK_{BH+}**-**Wert Base (H₂O)** | **pK_{BH+}**-**Wert Base (MeCN)** | **Abbindezeit** |
| Beispiel 1 | tert-Butylimino-tri(pyrrolidino)phosphoran | kein | 2,97 | 0,00 | | 28,4 ²⁾ | < 30 s |
| Beispiel 2 | 1-tert-Butyl-2,2,4,4,4-pentakis(dimethylamino)-2Λ⁵,4Λ⁵-catenadi(phosphazen) | kein | 3,49 | 0,00 | | 33,5 ²⁾ | < 30 s |
| Beispiel 3 | 1,8-Diazabicyclo[5.4.0]undec-7en | kein | 1,45 | 0,00 | 12 ⁴⁾ | 24,33 ²⁾ | 3'15" |
| Beispiel 4 | 1-Ethyl-2,2,4,4,4-pentakis(dimethylamino)-2Λ⁵,4Λ⁵-catenadi(phosphazen) | kein | 3,22 | 0,00 | | 32,9 ²⁾ | 4'30" |
| Beispiel 5 | 1,8-Diazabicyclo[5.4.0]undec-7en | Tris(hydroxymethyl)-methylamin | 1,45 | 1,37 | 12 ⁴⁾ | 24,33 ²⁾ | 4'45" |
| Beispiel 6 | 1-tert-Butyl-4,4,4-tris(dimethylamino)-2,2-bis-[tris(dimethylamino)-phosphoranylidenamino]-2A5,4A5-catenadi(phosphazen) | kein | 6,02 | 0,00 | | 41,9 ²⁾ | 5'00" |
| Beispiel 7 | tert-Octyliminotris(dimethylamino)phosphoran | kein | 2,76 | 0,00 | | 26,5 ²⁾ | 5'00" |
| Beispiel 8 | 1,8-Diazabicyclo[5.4.0]undec-7en | Natriumhydrogencarbonat | 1,45 | 1,37 | 12 ⁴⁾ | 24,33 ²⁾ | 5'00" |
| Beispiel 9 | 1,8-Diazabicyclo[5.4.0]undec-7en | Kaliumchlorid | 1,45 | 1,37 | 12 ⁴⁾ | 24,33 ²⁾ | 5'30" |
| Beispiel 10 | 1,1,3,3-Tetramethylguanidin | Kein | 0,50 | 0,00 | | 23,3 ²⁾ | 7'00" |
| Beispiel 11 | 2,8,9-Triisopropyl-2,5,8,9-tetraaza-1-phosphabicyclo[3.3.3]undecane | kein | 2,42 | 0,00 | | 33,63 ³⁾ | 8'00" |
| Beispiel 12 | 1,8-Diazabicyclo[5.4.0]undec-7en | Tetramethylammoniumhydrogenphthalat | 1,76 | 1,37 | 12 ⁴⁾ | 24,33 ²⁾ | 8'30" |
| Beispiel 13 | 1,5-Diazabicyclo[4.3.0]non-5-en | kein | 1,18 | 0,00 | | 23,89 ²⁾ | 9'00" |
| Beispiel 14 | 7-Methyl-1,5,7-triazabicyclo[4.4.0]dec-5-en | kein | 1,46 | 0,00 | | 25,44 ²⁾ | 9'30" |
| Beispiel 15 | 2-tert-Butyl-1,1,3,3,-tetramethylguanidin | kein | 1,63 | 0,00 | 14 ⁵⁾ | > 20 | 10'00" |
| Beispiel 16 | 1,5,7-Triazabicyclo[4.4.0]dec-5-en | kein | 1,32 | 0,00 | | 25,98 ²⁾ | 10'30" |
| Beispiel 17 | 1,8-Diazabicyclo[5.4.0]undec-7en | Dinatriumhydrogenphosphat | 1,45 | 1,37 | 12 ⁴⁾ | 24,33 ²⁾ | 11'00" |
| Beispiel 18 | 1,8-Diazabicyclo[5.4.0]undec-7en | Phthalsäure Monokaliumsalz | 1,45 | 1,37 | 12 ⁴⁾ | 24,33 ²⁾ | 11'30" |
| Vergleichsbeispiel 1 | 1,4-Diazabicydo[2.2.2]octan (DABCO) | kein | 0,68 | 0,00 | 8,8 ⁴⁾ | 18,3 ⁴⁾ | keine Reaktion |
| Vergleichsbeispiel 2 | Triethanolamin | kein | 0,90 | 0,00 | 7,77 ⁶⁾ | | keine Reaktion |
| Vergleichsbeispiel 3 | Tributylamin | kein | 1,12 | 0,00 | | 18,09 ²⁾ | keine Reaktion |

| | | | | | | | |
|---|---|---|---|---|---|---|---|
| ²⁾ Quelle: ChemFiles: Strong and Hindered Bases in Organic Synthesis, Vol.3, No. 1, Fluka ³⁾ Quelle: Topics in Current Chemistry, Vol. 223, John G. Verhade "P(RNCH2CH2)3N: Very Strong Non-Ionic Bases Useful in Organic Synthesis" ⁴⁾ Quelle: http://www.cem.msu.edu/∼reusch/VirtualText/suppmnt2.htm ⁵⁾ 50 %ig in Ethanol, Quelle: DHR Barton, J.D. Elliot, SD Gero, J.Chem.Soc.Perkin Trans.1 1982, 2085 ⁶⁾ Quelle: H. K. Hall, J. Am. Chem. Soc. 1957, 79, 5441 | | | | | | | |

**Tabelle 2**

| **Verarbeitungs-, Abbindezeiten und Stabilitäten von Abformmaterialien auf Basis Alkoxysilylpolyether im Vergleich zu den Beispielen 3 und 5 der** EP 1226808 A2 | | | |
|---|---|---|---|
| **Abformmaterial:** | **Verarbeitungszeit ¹⁾** | **Abbindezeit ²⁾** | **Stabilität: Abbindezeit nach einer Woche 60°C ³⁾** |
| Beispiel 3 | 1,00 min | 3,25 min | 3,25 min |
| | | | |
| Vergleichsbeispiel 4 Beispiel 3 und 5 EP 122 6808 A2 | 0,50 min | 5,00 | > 15,00 min |

| | | | |
|---|---|---|---|
| ¹⁾ gemäß ISO 4823 ²⁾ Die Abbindezeit wurde bestimmt über Rückstellung nach der Verformung nach ISO 4823 (Ausgabe 1992). ³⁾ Einlagerung in verschlossenen Schlauchbeuteln aus PE/AI/PE-Verbundfolie, Messung siehe unter ²⁾ | | | |

**Tabelle 3**

| **Erfindungsgemäße Beispiele für adstringierende bzw. antibakterielle Zu-sätze gemäß Beispiel 3** | | | | |
|---|---|---|---|---|
| **Katalysatorpaste** | **adstringierender bzw. antibakterieller Zusatz** | **Menge Zusatz (Teile)** | **Abbindezeit (min)** | **Stabilität Abbindezeit nach 1 Woche 60°C (min)** |
| 3a | ohne Zusatz | 0,0 | 3,25 | 3,25 |
| 3b | Triclosan | 0,6 | 3,00 | 3,50 |
| 3c | Epinephrin | 0,6 | 3,25 | 3,75 |
| 3d | Chlorhexidine | 0,6 | 3,50 | 3,50 |
| 3e | Hexetidine | 0,6 | 3,25 | 3,75 |

**Tabelle 4**

| **Anwendungstechnische Eigenschaften der Zusammensetzung gemäß Beispiel 3** | |
|---|---|
| | **Beispiel 3 1, 8-Diazabicyclo [5.4.0] undec- 7- en als Katalysatorbase** |
| **Konsistenz ¹⁾ Katalysatorkomponente B** | 37 mm |
| **Konsistenz ¹⁾ Basiskomponente A** | 34 mm |
| **Konsistenz der Mischung ²⁾** | 35 mm |
| **Lineare Maßänderung ³⁾** | - 0,6 % |
| **Shore A-Härte sofort nach Abbindeende ⁴⁾** | 55 |
| **Shore A-Härte sofort nach 15 Stunden Lagerung ⁴⁾** | 63 |
| **Kontaktwinkel ⁵⁾** | 40° |
| **Rückstellung nach der Verformung ⁶⁾** | 98,3 % |

| | |
|---|---|
| ¹⁾ in Anlehnung an ISO 4823, Konsistenz der Mischung, Belastungsgewicht 500 g, Belastungsdauer 15 s ²⁾ gemäß ISO 4823, Konsistenz der Mischung, Belastungsgewicht 1500 g, Belastungsdauer 5 s ³⁾ gemäß ISO 4823 ⁴⁾ gemäß DIN 53505 mit digitalem Shore-Härteprüfgerät Fa. Zwick ⁵⁾ gemessen nach der Methode des liegenden Tropfens, Kontaktwinkelmeßsystem Fa. Krüss G40, initialer Kontaktwinkel (Prüfkörperalter: Aufbringen des Tropfens 45 Sek. nach Mischbeginn), Messzeitpunkt: 30 Sek. nach Aufbringen des Tropfens, Verwendung von entmineralisiertem Wasser. ⁶⁾ gemäß ISO 4823 | |

## Patentansprüche

1. Kondensationsvernetzendes Dentalmaterial, insbesondere Dentalabformmaterial, enthaltend:
a) wenigstens einen alkoxysilylfunktionellen Polyether
b) wenigstens einen verstärkenden Füllstoff b₁) und/oder wenigstens einen nichtverstärkenden Füllstoff b₂),
c) ggf. Wasser und
d) wenigstens einen Katalysator
**dadurch gekennzeichnet, dass** der wenigstens eine alkoxysilylfunktionelle Polyether a) als dritte Struktureinheit jeweils an den terminalen Alkoxysilylgruppen angeordnete Alkylenspacer, welche besonders bevorzugt C₁-C₆-Alkylgruppen, ganz besonders bevorzugt C₁-C₃-Alkylgruppen und höchst besonders bevorzugt Ethylengruppen (C₂) und/oder Methylengruppen (C₁) sind, und
als vierte Struktureinheit 0 bis 8 mmol/g, besonders bevorzugt 0 bis 4 mmol/g, ganz besonders bevorzugt 0,02 bis 2 mmol/g und höchst bevorzugt 0,1 bis 0,4 mmol/g Urethangruppen und/oder 0 bis 8 mmol/g, besonders bevorzugt 0 bis 2 mmol/g, ganz besonders bevorzugt 0,02 bis 2 mmol/g und höchst bevorzugt 0,1 bis 0,4 mmol/g Harnstoffgruppen aufweist, wobei solche Alkoxysilylpolyether höchst besonders bevorzugt sind, die innerhalb der Polymerkette keine Urethan- und/oder Harnstoffgruppen enthalten und die an jedem Kettenende höchstens eine bzw. nicht mehr als eine Urethan und/oder Harnstoffgruppe und höchstens eine bzw. nicht mehr als eine Methylenspacergruppe tragen, wobei die einzelnen Struktureinheiten des wenigstens einen Polyethers a) vorzugsweise gemäß 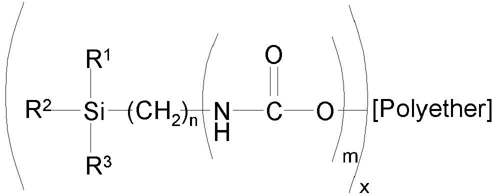 worin R¹, R² und R³ unabhängig voneinander Alkoxy-, Alkyl-, Aryl-, Aralkyl-, Alkylarylgruppen oder Wasserstoff, bevorzugt Butoxy-, Propoxy-, Ethoxy-, Methoxy-, Hexyl-, Pentyl-, Butyl-, Propyl-, Ethyl- oder Methylgruppen, besonders bevorzugt Ethoxy-, Methoxy-, Ethyl- oder Methylgruppen, sind mit der Maßgabe, dass mindestens einer der vorgenannten Reste, vorzugsweise zwei oder alle drei Reste, Alkoxygruppen sind, sowie
x=1 bis 6, bevorzugt x=2 bis 4 und ganz besonders bevorzugt x=2, n=1 bis 6, bevorzugt n=1 bis 3 und ganz besonders bevorzugt n=1 sowie m=0 oder 1, besonders bevorzugt m=1, ist/sind
und/oder 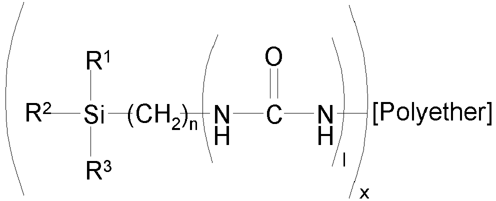 worin R¹, R² und R³ unabhängig voneinander Alkoxy-, Alkyl-, Aryl-, Aralkyl-, Alkylarylgruppen oder Wasserstoff, bevorzugt Butoxy-, Propoxy-, Ethoxy-, Methoxy-, Hexyl-, Pentyl-, Butyl-, Propyl-, Ethyl- oder Methylgruppen, besonders bevorzugt Ethoxy-, Methoxy-, Ethyl- oder Methylgruppen, sind mit der Maßgabe, dass mindestens einer der vorgenannten Reste, vorzugsweise zwei oder alle drei Reste, Alkoxygruppen sind, sowie
x=1 bis 6, bevorzugt x=2 bis 4 und ganz besonders bevorzugt x=2, n=1 bis 6, bevorzugt n=1 bis 3 und ganz besonders bevorzugt n=1 sowie l=0 oder 1, besonders bevorzugt l=1 ist/sind,
angeordnet sind,
dass der wenigstens eine verstärkende Füllstoffe b₁), als hochdisperser, aktiver Füllstoff eine BET-Oberfläche von wenigstens 50 m²/g und/oder einer Primärpartikelgröße von kleiner gleich 100 nm aufweist, und/oder der wenigstens eine nichtverstärkende Füllstoff b₂) zwingend eine BET-Oberfläche von weniger als 50 m²/g aufweist,
und dass der wenigstens eine Katalysator d) eine organische Base mit einem in Acetonitril gemessenen pK_{BH+}-Wert von mindestens 20 ist sowie wenigstens eine Struktureinheit gemäß der allgemeinen Formel I 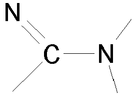 und/oder gemäß der allgemeinen Formel II 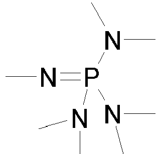 und/oder gemäß der allgemeinen Formel III 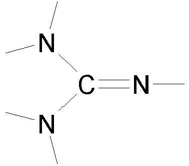 aufweist.

2. Kondensationsvernetzendes Zweikomponenten-Dentalmaterial, insbesondere Dentalabformmaterial, mit einer Komponente A enthaltend
a) wenigstens einen alkoxysilylfunktionellen Polyether und
b) wenigstens einen verstärkenden Füllstoff b₁) und/oder wenigstens einen nicht verstärkenden Füllstoff b₂)
sowie einer Komponente B enthaltend
c) Wasser und
d) wenigstens einen Katalysator,
**dadurch gekennzeichnet, dass** der wenigstens eine alkoxysilylfunktionelle Polyether a) als dritte Struktureinheit jeweils an den terminalen Alkoxysilylgruppen angeordnete Alkylenspacer, welche besonders bevorzugt C₁-C₆-Alkylgruppen, ganz besonders bevorzugt C₁-C₃-Alkylgruppen und höchst besonders bevorzugt Ethylengruppen (C₂) und/oder Methylengruppen (C₁) sind, und als vierte Struktureinheit 0 bis 8 mmol/g, besonders bevorzugt 0 bis 4 mmol/g, ganz besonders bevorzugt 0,02 bis 2 mmol/g und höchst bevorzugt 0,1 bis 0,4 mmol/g Urethangruppen und/oder 0 bis 8 mmol/g, besonders bevorzugt 0 bis 2 mmol/g, ganz besonders bevorzugt 0,02 bis 2 mmol/g und höchst bevorzugt 0,1 bis 0,4 mmol/g Harnstoffgruppen aufweist, wobei solche Alkoxysilylpolyether höchst besonders bevorzugt sind, die innerhalb der Polymerkette keine Urethan- und/oder Harnstoffgruppen enthalten und die an jedem Kettenende höchstens eine bzw. nicht mehr als eine Urethan und/oder Harnstoffgruppe und höchstens eine bzw. nicht mehr als eine Methylenspacergruppe tragen, wobei die einzelnen Struktureinheiten des wenigstens einen Polyethers a) vorzugsweise gemäß 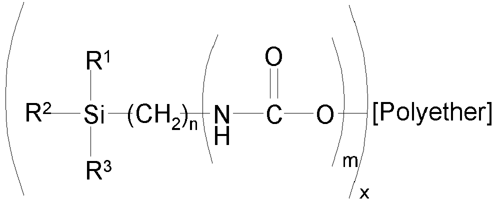 worin R¹, R² und R³ unabhängig voneinander Alkoxy-, Alkyl-, Aryl-, Aralkyl-, Alkylarylgruppen oder Wasserstoff, bevorzugt Butoxy-, Propoxy-, Ethoxy-, Methoxy-, Hexyl-, Pentyl-, Butyl-, Propyl-, Ethyl- oder Methylgruppen, besonders bevorzugt Ethoxy-, Methoxy-, Ethyl- oder Methylgruppen, sind mit der Maßgabe, dass mindestens einer der vorgenannten Reste, vorzugsweise zwei oder alle drei Reste, Alkoxygruppen sind, sowie
x=1 bis 6, bevorzugt x=2 bis 4 und ganz besonders bevorzugt x=2, n=1 bis 6, bevorzugt n=1 bis 3 und ganz besonders bevorzugt n=1 sowie m=0 oder 1, besonders bevorzugt m=1, ist/sind
und/oder 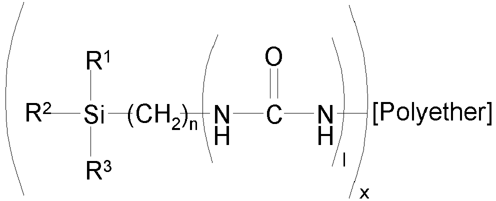 worin R¹, R² und R³ unabhängig voneinander Alkoxy-, Alkyl-, Aryl-, Aralkyl-, Alkylarylgruppen oder Wasserstoff, bevorzugt Butoxy-, Propoxy-, Ethoxy-, Methoxy-, Hexyl-, Pentyl-, Butyl-, Propyl-, Ethyl- oder Methylgruppen, besonders bevorzugt Ethoxy-, Methoxy-, Ethyl- oder Methylgruppen, sind mit der Maßgabe, dass mindestens einer der vorgenannten Reste, vorzugsweise zwei oder alle drei Reste, Alkoxygruppen sind, sowie
x=1 bis 6, bevorzugt x=2 bis 4 und ganz besonders bevorzugt x=2, n=1 bis 6, bevorzugt n=1 bis 3 und ganz besonders bevorzugt n=1 sowie l=0 oder 1, besonders bevorzugt l=1 ist/sind,
angeordnet sind,
dass der wenigstens eine verstärkende Füllstoffe b₁), als hochdisperser, aktiver Füllstoff eine BET-Oberfläche von wenigstens 50 m²/g und/oder einer Primärpartikelgröße von kleiner gleich 100 nm aufweist, und/oder der wenigstens eine nichtverstärkende Füllstoff b₂) zwingend eine BET-Oberfläche von weniger als 50 m²/g aufweist,
und der wenigstens eine Katalysator d) eine organische Base mit einem in Acetonitril gemessenen pK_{BH+}-Wert von mindestens 20 ist sowie wenigstens eine Struktureinheit gemäß der allgemeinen Formel I 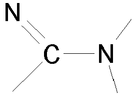 und/oder gemäß der allgemeinen Formel II 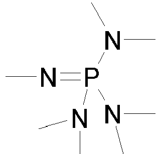 und/oder gemäß der allgemeinen Formel III 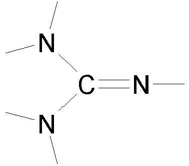 aufweist.

3. Dentalmaterial nach Anspruch 1 oder 2, **dadurch gekennzeichnet, dass** der wenigstens eine Katalysator d) eine organische Base mit einem in Acetonitril gemessenen pK_{BH+}-Wert von mindestens 21, bevorzugt mindestens 22 und besonders bevorzugt mindestens 23 ist.

4. Dentalmaterial nach einem der vorhergehenden Ansprüche, **dadurch gekennzeichnet, dass** die wenigstens eine Katalysatorbase d) in der Polyethermatrix, vorzugsweise Polytetrahydrofuran, Polyethylenglycol und besonders bevorzugt Polypropylenglycol und Mischungen und Copolymerisaten hiervon, eine hinreichend große Löslichkeit aufweist, um in einer auf die Gesamtmischung bezogenen Menge von 0,001 bis 1 mmol/g eingesetzt eine Aushärtung des Dentalmaterials bestimmt durch Rückstellung nach Verformung gemäß ISO 4823 (Ausgabe 1992) in weniger gleich 30 Minuten, bevorzugt weniger gleich 15 Minuten für eine Dubliermasse und in weniger gleich 15 Minuten, besonders bevorzugt weniger gleich 10 min, ganz besonders bevorzugt weniger gleich 7 min und höchst bevorzugt weniger gleich 6 min für eine dentale Abformmasse zu erzielen.

5. Dentalmaterial nach einem der vorhergehenden Ansprüche, **dadurch gekennzeichnet, dass** die wenigstens eine Katalysatorbase b) aus der aus 1,1,3,3-Tetramethylguanidin (TMG), 1,8- Diazabicyclo[5.4.0]undec-7-en (DBU), 1,5-Diazabicyclo[4.3.0]non-5-en (DBN), tert-Butylimino-tris(dimethylamino)-phosphoran, tert-Butylimino-tri(pyrrolidino)phosphoran, tert-Octylimino-tris(dimethylamino)phosphoran, 2-tert-Butylimino-2-diethylamino-1,3-dimethyl-perhydro- 1,3,2-diazaphosphorin, 2-tert-Butylimino-2-diethylamino-1,3-dimethyl-perhydro-1,3,2-diazaphosphorin auf Polystyrol, 1-tert-Butyl-2,2,4,4,4-pentakis(diethylamino)-2A5, 4A5-catenadi(phosphazen), 1-Ethyl-2,2,4,4,4-pentakis(diethylamino)-2A5, 4A5-catenadi(phosphazen), 1-tert-Butyl-4,4,4-tris(dimethylamino)-2,2-bis[tris(dimethylamino)-phosphor-anylidenamino]-2A⁵, 4Λ⁵-catenadi(phosphazen), 1-tert-Octyl-4,4,4-tris(dimethylamino)-2,2-bis[tris-(dimethylamino)-phosphor-anylidenamino]-2Λ⁵,4Λ⁵-catenadi(phosphazen), 2,8,9-Triisobutyl-2,5,8,9-tetraaza-1-phosphabicyclo[3.3.3]undecan, 2,8,9-Tri-isopropyl-2,5,8,9-tetraaza-1-phosphabicyclo[3.3.3]undecan, 2,8,9-Trimethyl-2,5,8,9-tetraaza-1-phosphabicyclo[3.3.3]undecan, 2-tert-Butyl-1,1,3,3-tetra-methylguanidin, 1,8-Bis(tetramethylguanidino)naphthalen (TMGN),1,5,7-Triazabicyclo(4.4.0)dec-5-en, 7-Methyl-1,5,7-triazabicyclo(4.4.0)dec-5-en, 1,5-Diazabicyclo[4.3.0)non-5-en und 3,3,6,9,9-Pentamethyl-2,10-diazabicyclo-(4.4.0)dec-1-en bestehenden Gruppe ausgewählt ist.

6. Dentalmaterial nach einem der vorhergehenden Ansprüche, **dadurch gekennzeichnet, dass** dieses des Weiteren wenigstens ein Puffersalz e) enthält, welches vorzugsweise aus der aus Alkalimetallhydrogencarbonat, Dialkalihydrogenphosphat, Tris(hydroxymethyl)aminomethan, Phthalsäure-Monoalkalisalz, Phthalsäuremonotetramethylammoniumsalz, Ammoniumsalzen von Aminen, cyclischen Aminen, Amiden und cyclischen Amiden, N-(2-Acetamido)-2-aminoethansulfonsäure, N-(2-Acetamido)iminodiessigsäure, β-Alanin, Ameisensäure (98-100 %), 2-Amino-2-methyl-1-propanol, Barbital, Barbital-Natrium, Bernsteinsäure, N,N-Bis-(2-hydroxyethyl)-2-aminoethansulfonsäure, N,N-Bis(2-hydroxyethyl)-glycin, 2,2-Bis-(Hydroxyethyl)-(iminotris)-(hydroxyamethyl)methan, Borsäure, CHAPS (3-[(3-Cholamidoproypl)-dimethyl-ammonio]-propan-sulfonat), Citronensäure-Monohydrat, 2-(Cyclo-hexylamino)-ethansulfonsäure, Diethanolamin, Essigsäure (Eisessig; 100 %), Glycin, Glycylglycin, 2-[4-2-Hydroxyethyl)-1-piperazinyl]-ethansulfonsäure, 2-[4-(2-Hydroxyethyl)-1-piperazinyl]-ethansulfonsäure Natriumsalz, 3-[4-(2-Hydroxyethyl)-1-piperazinyl]-propansulfonsäure, Imidazol, Kaliumdihydrogenphosphat, di-Kaliumhydrogenphosphat-Trihydrat, Kaliumhydrophenphthalat, 1-Methylimidazol, 2-Morpholinoethansulfonsäure-Monohydrat, 3-Morpho-linopropansulfonsäure, Natriumacetat-Trihydrat, Natriumcarbonat, tri-Natriumcitrat-Dihydrat, Natriumdihydrogenphosphat-Monohydrat, tretra-Natriumdiphosphat-Decahydrat, Natriumhydrogencarbonat, di-Natrium-hydrogenphosphat-Dihydrat, di-Natriumoxalat, di-Natriumtetraborat-Decahydrat, Piperazin-1,4-bis(2-ethansulfonsäure), Piperazin-1,4-bis-(2-ethansulfonsäure) Mononatriumsalz, Triethanolamin, Triethanolaminhydrochlorid, 2,4,6-Tri-methylpyridin, Tris(hydroxymethyl)-aminomethan, Tris(hydroxymethyl)-aminomethanhydrochlorid, N-[Tris(hydroxymethyl)-methyl]-2-amino-ethansulfon-säure und N-[Tris(hydroxymethyl)-methyl]-3-aminopropansulfonsäure bestehenden Gruppe ausgewählt ist.

7. Dentalmaterial nach einem der vorhergehenden Ansprüche, **dadurch gekennzeichnet, dass** dieses des Weiteren wenigstens einen Wasserfänger d) enthält, welcher vorzugsweise Vinyltrimethoxysilan, N-Trimethoxysilylmethyl-O-methylcarbamat und/oder eine Verbindung der folgenden Formel: 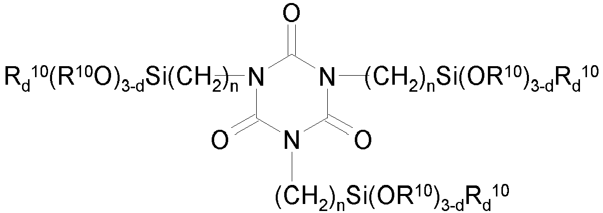
worin n = 1 bis 6, bevorzugt n = 1 oder 3, besonders bevorzugt n = 1,
d = 0 oder 1, und
R¹⁰ = ein linearer oder verzweigter C₁-C₃₀-Alkylrest, in dem die Wasserstoffatome teilweise durch Halogenatome, OH-, NH₂-, NO₂- oder auch weitere C₁-C₆-Alkylreste substituiert sein können, sind,
ist.

8. Dentalmaterial nach einem der vorhergehenden Ansprüche, **dadurch gekennzeichnet, dass** dieses des Weiteren wenigstens einen Pastenbildner h) enthält, welcher vorzugsweise aus der aus Polyethern, Polyvinylpyrrolidonen, Polyurethanen, Polyestern, Wachsen, Vaseline, Paraffinölen, Siliconölen, mehrwertigen Alkoholen, Propylenglycolen, Polypropylenglycolen, Ethylenglycolen, Polyethylenglycolen, Copolymerisaten aus N-Vinylpyrrolidon und Vinylacetat, Carboxymethyl-, Methyl-, Hydroxyethyl-, Hydroxypropyl-Cellulose, Polysacchariden und Poly(meth)acrylsäuren bestehenden Gruppe ausgewählt ist.

9. Dentalmaterial nach einem der vorhergehenden Ansprüche, **dadurch gekennzeichnet, dass** dieses des Weiteren wenigstens einen Stabilisator u) enthält, welcher vorzugsweise aus der aus polymerem Trimethyl-dihydrochinolin, Diphenylderivaten, Phenothiazin, Phenyl-α-naphthylamin, 4, 4'-Methylen-bis-2,6-di-tert.-butylphenol, Butylhydroxytoluol, Butylhydroxyanisol (BHA) und Methoxy-phenol (Hydroxyanisol) bestehenden Gruppe ausgewählt ist.

10. Dentalmaterial nach einem der vorhergehenden Ansprüche, **dadurch gekennzeichnet, dass** dieses neben einer oder mehreren Basen gemäß einem der Ansprüche 1 bis 6 keinen weiteren Katalysator, insbesondere keine metallorganischen Verbindungen, keine Schwermetallcarboxylatsalze, keine Amine mit einem pk_{BH+}-Werten von weniger als 20 und keine freien Säuren, enthält.

11. Dentalmaterial nach einem der vorhergehenden Ansprüche, **dadurch gekennzeichnet, dass** dieses, bezogen auf die Gesamtmischung, 0,001 bis 1,0 mmol/g, besonders bevorzugt 0,001 bis 0,5, ganz besonders bevorzugt 0,001 bis 0,1 mmol/g und höchst bevorzugt 0,005 bis 0,05 mmol/g wenigstens einer organischen Base als Katalysator d) enthält.

12. Dentalmaterial nach Anspruch 13, **dadurch gekennzeichnet, dass** n gleich 1 ist.

13. Dentalmaterial nach einem der vorhergehenden Ansprüche, **dadurch gekennzeichnet, dass** der wenigstens eine Polyether a) eine aus der aus Polytetrahydrofuran, Polyethylenglycol, Polypropylenglycol, Copolymerisaten hiervon und Mischungen hiervon bestehenden Gruppe ausgewählte Struktureinheit enthält.

14. Mischung erhältlich durch Vermischen der Komponenten A und B des Zweikomponenten-Dentalmaterials nach einem der Ansprüche 2 bis 13, **dadurch gekennzeichnet, dass** die Basiskomponente A mit der Katalysatorkomponente B in einem Verhältnis von 1:1 bis 20:1, besonders bevorzugt von 1:1 bis 10:1 und ganz besonders bevorzugt von 1:1, 2:1, 4:1, 5:1 und 10:1 vermischt wird.

15. Verwendung eines Dentalmaterials nach einem der Ansprüche 1 bis 14 in der Dentalmedizin und/oder Dentaltechnik.

## Claims

1. Condensation-curing dental material, in particular dental impression material, containing
a) at least one alkoxysilyl-functional polyether
b) at least one reinforcing filler b₁) and/or at least one non-reinforcing filler b₂)
(c) water, where appropriate and
(d) at least one catalyst
**characterized in that** the at least one alkoxysilyl-functional polyether a) features as a third structural unit alkylene spacers which are arranged in each case at the terminal alkoxysilyl groups and which are particularly preferably C₁-C₆-alkyl groups, very particularly preferably C₁-C₃-alkyl groups and most particularly preferably ethylene groups (C₂) and/or methylene groups (C₁), and as a fourth structural unit 0 to 8 mmol/g, particularly preferably 0 to 4 mmol/g, very preferably 0.02 to 2 mmol/g and most preferably 0.1 to 0.4 mmol/g of urethane groups and/or 0 to 8 mmol/g, particularly preferably 0 to 2 mmol/g, very preferably 0,02 to 2 mmol/g and most preferably 0.1 to 0.4 mmol/g of urea groups, wherein those alkoxysilyl polyethers are most preferred, which do not contain any urethane and/or urea groups within the polymer chain and which contain not more than one urethane and/or urea group at each chain end and which contain not more than one methylene spacer group, wherein the individual structural units of the at least one polyether a) is preferably according to 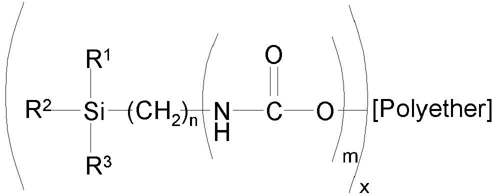 in which R¹, R² and R³ independently of one another are alkoxy, alkyl, aryl, aralkyl, alkylaryl groups or hydrogen, preferably butoxy, propoxy, ethoxy, me-thoxy, hexyl, pentyl, butyl, propyl, ethyl or methyl groups, particularly preferably ethoxy, methoxy, ethyl or methyl groups, with the proviso that at least one of the above-mentioned substituents, preferably two or all three substituents, are alkoxy groups, and
x=1 to 6, preferably x=2 to 4 and most preferably x=2, n=1 to 6, preferably n=1 to 3 and most preferably n=1 and m=0 or 1, especially preferably m=1, is/are
and/or 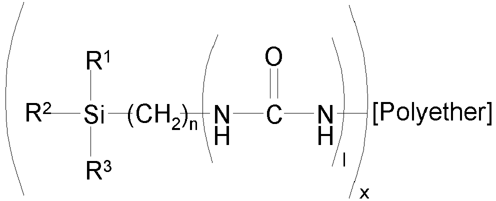 in which R¹, R² and R³ independently of one another are alkoxy, alkyl, aryl, aralkyl, alkylaryl groups or hydrogen, preferably butoxy, propoxy, ethoxy, me-thoxy, hexyl, pentyl, butyl, propyl, ethyl or methyl groups, particularly preferably ethoxy, methoxy, ethyl or methyl groups, with the proviso that at least one of the above-mentioned radicals, preferably two or all three radicals, are alkoxy groups, and
x=1 to 6, preferably x=2 to 4 and most preferably x=2, n=1 to 6, preferably n=1 to 3 and more preferably n=1 and l=0 or 1, most preferably l=1,
**in that** the at least one reinforcing filler b₁) as a highly dispersed, active filler, has a BET surface area of at least 50 m²/g and/or a primary particle size of less than or equal to 100 nm, and/or the at least one non-reinforcing filler b₂) necessarily has a BET surface area of less than 50 m²/g,
and that the at least one catalyst d) is an organic base with a pK_{BH}⁺ value of at least 20, measured in acetonitrile, and shows at least one structural unit according to the general formula I 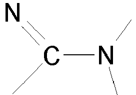 and/or according to the general formula II 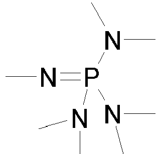 and/or according to the general formula III

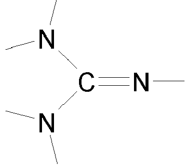

2. Condensation-curing two-component dental material, in particular dental impression material, comprising a component A containing
a) at least one alkoxysilyl functional polyether; and
b) at least one reinforcing filler b₁) and/or at least one non-reinforcing filler b₂)
and a component B containing
c) water; and
d) at least one catalyst,
**characterized in that** the at least one alkoxysilyl-functional polyether a) features as a third structural unit, alkylene spacers arranged in each case at the terminal alkoxysilyl groups, which are particularly preferably C₁-C₆-alkyl groups, very particularly preferably C₁-C₃-alkyl groups and most preferably ethylene groups (C₂) and/or methylene groups (C₁), and as a fourth structural unit 0 to 8 mmol/g, particularly preferably 0 to 4 mmol/g, very particularly preferably 0.02 to 2 mmol/g and most preferably 0.1 to 0.4 mmol/g of urethane groups and/or 0 to 8 mmol/g, particularly preferably 0 to 2 mmol/g, very particularly preferably 0.02 to 2 mmol/g and most preferably 0.1 to 0.4 mmol/g of urea groups, wherein those alkoxysilyl polyethers are most preferred, which do not contain any urethane and/or urea groups within the polymer chain and which contain not more than one urethane and/or urea group at each chain end and which contain not more than one methylene spacer group, wherein the individual structural units of the at least one polyether a) is preferably according to 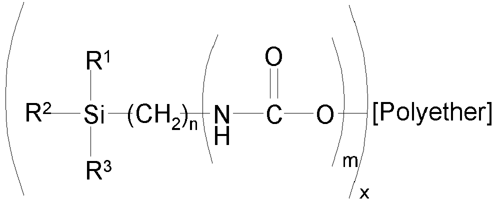 in which R¹, R² and R³ independently of one another are alkoxy, alkyl, aryl, aralkyl, alkylaryl groups or hydrogen, preferably butoxy, propoxy, ethoxy, me-thoxy, hexyl, pentyl, butyl, propyl, ethyl or methyl groups, particularly preferably ethoxy, methoxy, ethyl or methyl groups, with the proviso that at least one of the above-mentioned radicals, preferably two or all three radicals, are alkoxy groups, and
x=1 to 6, preferably x=2 to 4 and most preferably x=2, n=1 to 6, preferably n=1 to 3 and most preferably n=1 and m=0 or 1, especially preferably m=1, is/are
and/or 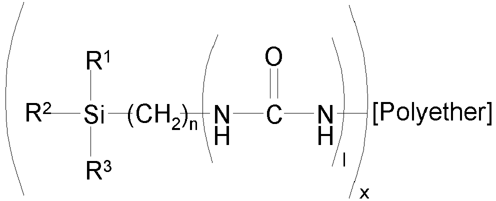 in which R¹, R² and R³ independently of one another are alkoxy, alkyl, aryl, aralkyl, alkylaryl groups or hydrogen, preferably butoxy, propoxy, ethoxy, me-thoxy, hexyl, pentyl, butyl, propyl, ethyl or methyl groups, particularly preferably ethoxy, methoxy, ethyl or methyl groups, with the proviso that at least one of the above-mentioned radicals, preferably two or all three radicals, are alkoxy groups, and
x=1 to 6, preferably x=2 to 4 and very especially x=2, n=1 to 6, preferably n=1 to 3 and very especially n=1 and l=0 or 1, especially preferably l=1,
**in that** the at least one reinforcing filler b₁) as a highly dispersed, active filler, has a BET surface area of at least 50 m²/g and/or a primary particle size of less than or equal to 100 nm, and/or the at least one non-reinforcing filler b₂) necessarily has a BET surface area of less than 50 m²/g,
and that the at least one catalyst d) is an organic base with a pK_{BH}⁺ value of at least 20, measured in acetonitrile, and shows at least one structural unit according to the general formula I 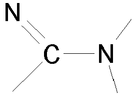 and/or according to the general formula II 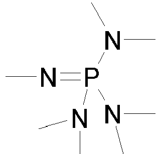 and/or according to the general formula III

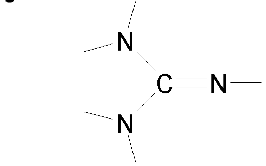

3. Dental material according to claim 1 or 2, **characterized in that** the at least one catalyst d) is an organic base with a pK_{BH}⁺ value measured in acetonitrile of at least 21, preferably at least 22 and most preferably at least 23.

4. Dental material according to any of the preceding claims, **characterized in that** the at least one catalyst base d) in the polyether matrix, preferably polytetrahydrofuran, polyethylene glycol and particularly preferably polypropylene glycol and mixtures and copolymers thereof, has a sufficiently high solubility to be present in an amount of 0,001 to 1 mmol/g relative to the total mixture, achieving a hardening of the dental material determined by recovery after deformation according to ISO 4823 (1992 edition) in less than or equal to 30 minutes, preferably less than or equal to 15 minutes for a duplicating material and in less than or equal to 15 minutes, particularly preferably less than or equal to 10 minutes, very particularly preferably less than or equal to 7 minutes and most preferably less than or equal to 6 minutes for a dental impression material

5. Dental material according to any of the preceding claims, **characterized in that** the at least one catalyst base d) is selected from the group consisting of 1,1,3,3-tetramethylguanidine (TMG), 1,8-diazabicyclo[5.4.0]undec-7-ene (DBU), 1,5-diazabicyclo[4.3.0]non-5-ene (DBN), tert-butylimino-tris(dimethylamino)-phosphorane, tert-butylimino-tri(pyrrolidino)phosphorane, tert-octylimino-tris(dimethylamino)phosphorane, 2-tert-butylimino-2-diethylamino-1, 3-dimethyl-perhydro- 1,3,2- diazaphosphorine, 2-tert-butylimino-2-diethylamino-1,3-dimethylperhydro- 1,3,2-diazaphosphorine on polystyrene, 1-tert-butyl-2,2,4,4,4-pentakis(diethylamino)-2Λ⁵, 4Λ⁵-catenadi(phosphazene), 1-ethyl-2,2,4,4,4-pentakis(diethylamino)-2Λ⁵, 4Λ⁵-catenadi(phosphazene), 1-tert-butyl-4,4,4-tris(dimethylamino)-2,2-bis[tris(dimethylamino)-phosphor-anylidenamino]-2Λ5, 4Λ5-catenadi(phosphazene), 1-tert-octyl-4,4,4-tris(dimethylamino)-2,2-bis[tris-(dimethylamino)-phosphor-anylidenamino]-2Λ⁵,4Λ⁵-catenadi(phosphazene), 2,8,9-trisobutyl-2,5,8,9-tetraaza-1-phosphabicyclo[3.3.3]undecane, 2,8,9-tri-isopropyl-2,5,8,9-tetraaza-1-phosphabicyclo[3.3.3]undecane, 2,8,9-trimethyl-2,5,8,9-tetraaza-1-phosphabicyclo[3.3.3]undecane, 2-tert-butyl-1,1,3,3-tetramethylguanidine, 1,8-bis(tetramethylguanidino)naphthalene (TMGN), 1,5,7-triazabicyclo[4. 4.0]dec-5-ene, 7-methyl-1,5,7-triazabicyclo[4.4.0]dec-5-ene, 1,5-diazabicyclo[4.3.0]non-5-ene and 3,3,6,9,9-pentamethyl-2,10-diazabicyclo-[4.4.0]dec-1-ene.

6. Dental material according any of the preceding claims, **characterized in that** it further comprises at least one buffer salt e) preferably selected from the group consisting of alkali metal hydrogen carbonate, dialkali hydrogen phosphate, tris(hydroxymethyl)aminomethane, phthalic acid monoalkali salt, phthalic acid monotetramethylammonium salt, ammonium salts of amines, cyclic amines, amides and cyclic amides, N-(2-acetamido)-2-aminoethanesulfonic acid, N-(2-acetamido)iminodiacetic acid, β-alanine, formic acid (98-100%), 2-amino-2-methyl-1-propanol, barbitol, barbitol sodium, succinic acid, N,N-bis(2-hydroxyethyl)-2-aminoethanesulfonic acid, N,N-bis(2-hydroxyethyl)-glycine, 2,2-bis-(hydroxyethyl)-(iminotris)-(hydroxymethyl)methane, boric acid, CHAPS (3-[(3-cholamidopropyl)dimethyl-ammonio]-propane sulfonate), citric acid monohydrate, 2-(cyclo-hexylamino)-ethanesulfonic acid, diethanolamine, acetic acid (glacial acetic acid; 100 %), glycine, glycylglycine, 2-[4-2-hydroxyethyl)-1-piperazinyl]-ethanesulfonic acid, 2-[4-(2-hydroxyethyl)-1-piperazinyl]-ethanesulfonic acid sodium salt, 3-[4-(2-hydroxyethyl)-1-piperazinyl]-propanesulfonic acid, imidazole, potassium dihydrogen phosphate, di-potassium hydrogen phosphate trihydrate, potassium hydrophenephthalate, 1-methylimidazole, 2-morpholinoethanesulfonic acid monohydrate, 3-morpho-linopropanesulfonic acid, sodium acetate trihydrate, sodium carbonate, tri-sodium citrate dihydrate, sodium dihydrogen phosphate monohydrate, tetra-sodium diphosphate decahydrate, sodium hydrogen carbonate, di-sodium hydrogen phosphate dihydrate, di-sodium oxalate, di-sodium tetraborate decahydrate, piperazine-1,4-bis(2-ethanesulfonic acid), piperazine-1,4-bis(2-ethanesulfonic acid) monosodium salt, triethanolamine, triethanolamine hydrochloride, 2,4,6-trimethylpyridine, tris(hydroxymethyl)aminomethane, tris(hydroxymethyl)aminomethane hydrochloride, N-[tris(hydroxymethyl)methyl]-2-amino-ethanesulfonic acid and N-[tris(hydroxymethyl)methyl]-3-aminopropanesulfonic acid.

7. Dental material according to any of the preceding claims, **characterized in that** it further comprises at least one water scavenger d) which preferably comprises vinyltrimethoxysilane, N-trimethoxysilylmethyl-O-methylcarbamate and/or a compound of the following formula: 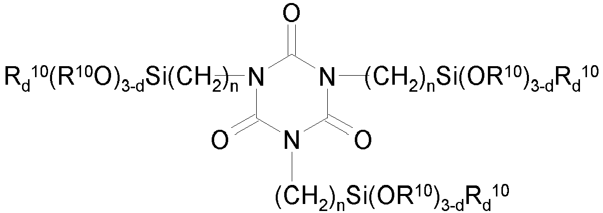 wherein n = 1 to 6, preferably n = 1 or 3, particularly preferably n = 1, d = 0 or 1, and
R¹⁰ = a linear or branched C₁-C₃₀-alkyl radical in which the hydrogen atoms may be partially substituted by halogen atoms, OH, NH₂, NO₂ or other C₁-C₆-alkyl radicals.

8. Dental material according to any of the preceding claims, **characterized in that** it further comprises at least one paste-forming agent h) preferably selected from the group consisting of polyethers, polyvinylpyrrolidones, polyurethanes, polyesters, waxes, petroleum jellies, paraffin oils, silicone oils, polyhydric alcohols, propylene glycols, polypropylene glycols, ethylene glycols, polyethylene glycols, copolymers of N-vinylpyrrolidone and vinylacetate, carboxymethyl-methyl-hydroxyethyl-hydroxypropyl-cellulose, polysaccharides and poly(meth)acrylic acids.

9. Dental material according to any of the preceding claims, **characterized in that** it further contains at least one stabilizer u) preferably selected from the group consisting of polymeric trimethyl-dihydroquinoline, diphenyl derivatives, phenothiazine, phenyl-a-naphthylamine, 4,4'-methylene-bis-2,6-di-tert-butylphenol, butylhydroxytoluene, butylhydroxyanisole (BHA) and methoxy-phenol (hydroxyanisole).

10. Dental material according to any of the preceding claims, **characterized in that**, apart from one or more bases according to one of the claims 1 to 6, it does not contain any other catalyst, in particular no metallorganic compounds, no heavy metal carboxylate salts, no amines with a pk_{BH}⁺ value of less than 20 and no free acids.

11. Dental material according to any of the preceding claims, **characterized in that** it contains, relative to the total mixture, 0.001 to 1.0 mmol/g, particularly preferably 0.001 to 0.5, very particularly preferably 0.001 to 0.1 mmol/g and most preferably 0.005 to 0.05 mmol/g of at least one organic base as catalyst d).

12. Dental material according to claim 13, **characterized in that** n is equal to 1.

13. Dental material according to any of the preceding claims, **characterized in that** the at least one polyether a) contains a structural unit selected from the group consisting of polytetrahydrofuran, polyethylene glycol, polypropylene glycol, copolymers thereof and mixtures thereof.

14. Mixture obtainable by mixing components A and B of the two-component dental material according to any one of Claims 2 to 13, **characterized in that** base component A is mixed with catalyst component B in a ratio of 1:1 to 20:1, particularly preferably of 1:1 to 10:1 and very particularly preferably of 1:1, 2:1, 4:1, 5:1 and 10:1.

15. Use of a dental material according to one of claims 1 to 14 in dentistry and/or dental technology.

## Revendications

1. Matériau dentaire subissant une réticulation par condensation, notamment matériau à empreintes dentaires, contenant:
a) au moins un polyéther aux fonctions alkoxysilyles
b) au moins une charge renforçante b₁) et/ou au moins une charge non-renforçante b₂),
c) le cas échéant, de l'eau, et
d) au moins un catalyseur
**caractérisé en ce que** l'au moins un polyéther aux fonctions alkoxysilyles a) comporte, en tant que troisième unité structurelle, des groupes d'espacement de type alkylène qui sont disposés sur chacun des groupes alkoxysilyles terminaux, s'agissant avec une préférence particulière de groupes alkyles en C₁-C₆, avec une préférence toute particulière de groupes alkyles en C₁-C₃, et avec la plus grande préférence de groupes éthylène (C₂) et/ou de groupes méthylène (C₁), et
en tant que quatrième unité structurelle, 0 à 8 mmol/g, avec une préférence particulière 0 à 4 mmol/g, avec une préférence toute particulière 0,02 à 2 mmol/g, et avec la plus grande préférence 0,1 à 0,4 mmol/g, de groupes uréthane et/ou 0 à 8 mmol/g, avec une préférence particulière 0 à 2 mmol/g, avec une préférence toute particulière 0,02 à 2 mmol/g, et avec la plus grande préférence 0,1 à 0,4 mmol/g, de groupes urée les polyéthers aux fonctions alkoxysilyles qui ne contiennent pas de groupes uréthane et/ou urée au sein de la chaîne polymérique et qui portent, à chaque extrémité de chaîne, au maximum un ou pas plus d'un groupe uréthane et/ou urée, et au maximum un ou pas plus d'un groupe d'espacement de type méthylène, étant notamment les plus préférés, chacune des unités structurelles de l'au moins un polyéther a) étant préférentiellement disposée selon 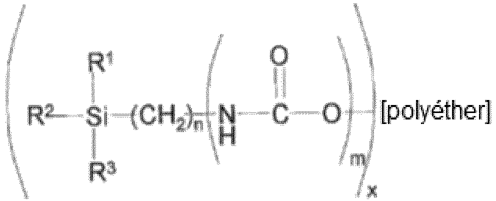 R¹, R² et R³ étant indépendamment l'un de l'autre des groupes alkoxy, alkyle, aryle, aralkyle, alkylaryle ou de l'hydrogène, s'agissant préférentiellement de groupes butoxy, propoxy, éthoxy, méthoxy, hexyle, pentyle, butyle, propyle, éthyle ou méthyle, avec une préférence particulière de groupes éthoxy, méthoxy, éthyle ou méthyle, à condition qu'au moins un des radicaux précités, de préférence deux ou l'ensemble des trois radicaux, corresponde des groupes alkoxy, ainsi que
x = 1 à 6, préférentiellement x = 2 à 4 et, avec une préférence toute particulière x = 2, n = 1 à 6, préférentiellement n = 1 à 3 et, avec une préférence toute particulière n = 1 ainsi que m = 0 ou 1, avec une préférence particulière m = 1,
et/ou 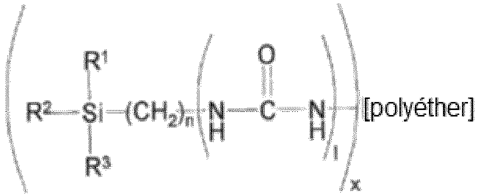 R¹, R² et R³ étant indépendamment l'un de l'autre des groupes alkoxy, alkyle, aryle, aralkyle, alkylaryle ou de l'hydrogène, s'agissant préférentiellement de groupes butoxy, propoxy, éthoxy, méthoxy, hexyle, pentyle, butyle, propyle, éthyle ou méthyle, avec une préférence particulière de groupes éthoxy, méthoxy, éthyle ou méthyle, à condition qu'au moins un des radicaux précités, de préférence deux ou l'ensemble des trois radicaux, corresponde à des groupes alkoxy, ainsi que
x = 1 à 6, préférentiellement x = 2 à 4 et, avec une préférence toute particulière x = 2, n = 1 à 6, préférentiellement n = 1 à 3 et, avec une préférence toute particulière n = 1 ainsi que 1 = 0 ou 1, avec une préférence particulière 1 = 1,
**que** l'au moins une charge renforçante b₁) présente, en tant que charge active à haut degré de dispersion, une surface BET d'au moins 50 m²/g et/ou une taille des particules primaires inférieure ou égale à 100 nm, et/ou l'au moins une charge non-renforçante b₂) présente obligatoirement une surface BET inférieure à 50 m²/g, et que l'au moins un catalyseur d) est une base organique ayant une valeur pK_{BH+}, mesurée dans l'acétonitrile, d'au moins 20, et comporte au moins une unité structurelle répondant à la formule générale I 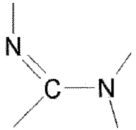 et/ou répondant à la formule générale II 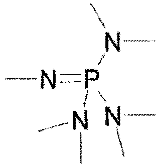 et/ou répondant à la formule générale III

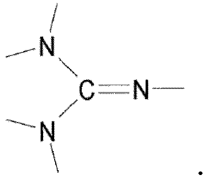

2. Matériau dentaire à deux composants subissant une réticulation par condensation, notamment matériau à empreintes dentaires, avec un composant A contenant
a) au moins un polyéther aux fonctions alkoxysilyles, et
b) au moins une charge renforçante b₁) et/ou au moins une charge non-renforçante b₂)
ainsi qu'un composant B contenant
c) de l'eau, et
d) au moins un catalyseur,
**caractérisé en ce que** l'au moins un polyéther aux fonctions alkoxysilyles a) comporte, en tant que troisième unité structurelle, des groupes d'espacement de type alkylène qui sont disposés sur chacun des groupes alkoxysilyles terminaux, s'agissant avec une préférence particulière de groupes alkyles en C₁-C₆, avec une préférence toute particulière de groupes alkyles en C₁-C₃, et avec la plus grande préférence de groupes éthylène (C₂) et/ou de groupes méthylène (C₁), et en tant que quatrième unité structurelle, 0 à 8 mmol/g, avec une préférence particulière 0 à 4 mmol/g, avec une préférence toute particulière 0,02 à 2 mmol/g, et avec la plus grande préférence 0,1 à 0,4 mmol/g, de groupes uréthane et/ou 0 à 8 mmol/g, avec une préférence particulière 0 à 2 mmol/g, avec une préférence toute particulière 0,02 à 2 mmol/g, et avec la plus grande préférence 0,1 à 0,4 mmol/g, de groupes urée, les polyéthers aux fonctions alkoxysilyles qui ne contiennent pas de groupes uréthane et/ou urée au sein de la chaîne polymérique et qui portent, à chaque extrémité de chaîne, au maximum un ou pas plus d'un groupe uréthane et/ou urée, et au maximum un ou pas plus d'un groupe d'espacement de type méthylène, étant notamment les plus préférés, chacune des unités structurelles de l'au moins un polyéther a) étant préférentiellement disposée selon 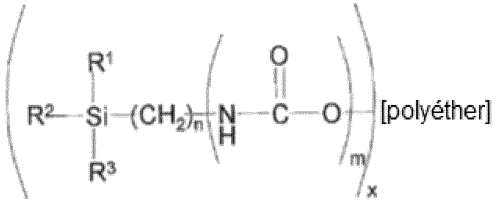 R¹, R² et R³ étant indépendamment l'un de l'autre des groupes alkoxy, alkyle, aryle, aralkyle, alkylaryle ou de l'hydrogène, s'agissant préférentiellement de groupes butoxy, propoxy, éthoxy, méthoxy, hexyle, pentyle, butyle, propyle, éthyle ou méthyle, avec une préférence particulière de groupes éthoxy, méthoxy, éthyle ou méthyle, à condition qu'au moins un des radicaux précités, de préférence deux ou l'ensemble des trois radicaux, corresponde à des groupes alkoxy, ainsi que
x = 1 à 6, préférentiellement x = 2 à 4 et, avec une préférence toute particulière x = 2, n = 1 à 6, préférentiellement n = 1 à 3 et, avec une préférence toute particulière n = 1 ainsi que m = 0 ou 1, avec une préférence particulière m = 1,
et/ou 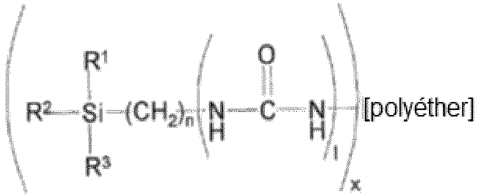 R¹, R² et R3 étant indépendamment l'un de l'autre des groupes alkoxy, alkyle, aryle, aralkyle, alkylaryle ou de l'hydrogène, s'agissant préférentiellement de groupes butoxy, propoxy, éthoxy, méthoxy, hexyle, pentyle, butyle, propyle, éthyle ou méthyle, avec une préférence particulière de groupes éthoxy, méthoxy, éthyle ou méthyle, à condition qu'au moins un des radicaux précités, de préférence deux ou l'ensemble des trois radicaux, corresponde à des groupes alkoxy, ainsi que
x = 1 à 6, préférentiellement x = 2 à 4 et, avec une préférence toute particulière x = 2, n = 1 à 6, préférentiellement n = 1 à 3 et, avec une préférence toute particulière n = 1 ainsi que l = 0 ou 1, avec une préférence particulière l = 1,
**que** l'au moins une charge renforçante b₁) présente, en tant que charge active à haut degré de dispersion, une surface BET d'au moins 50 m²/g et/ou une taille des particules primaires inférieure ou égale à 100 nm, et/ou l'au moins une charge non-renforçante b₂) présente obligatoirement une surface BET inférieure à 50 m²/g, et que l'au moins un catalyseur d) est une base organique ayant une valeur pK_{BH+}, mesurée dans l'acétonitrile, d'au moins 20, et comporte au moins une unité structurelle répondant à la formule générale I 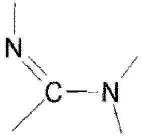 et/ou répondant à la formule générale II 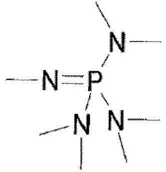 et/ou répondant à la formule générale III

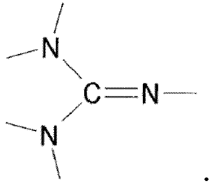

3. Matériau dentaire selon les revendications 1 ou 2, **caractérisé en ce que** l'au moins un catalyseur d) est une base organique ayant une valeur pK_{BH+}, mesurée dans l'acétonitrile, d'au moins 21, avec une préférence particulière d'au moins 22, et avec une préférence particulière d'au moins 23.

4. Matériau dentaire selon l'une des revendications précédentes, **caractérisé en ce que** l'au moins une base de catalyseur d) présente une solubilité dans la matrice de polyéther, s'agissant préférentiellement de polytétrahydrofurane, de polyéthylèneglycol et, avec une préférence particulière, de polypropylèneglycol et de mélanges et produits de copolymérisation de celui-ci, qui est suffisante pour obtenir, lorsqu'elle est employée dans une quantité comprise entre 0,001 et 1 mmol/g, par rapport au mélange dans son intégralité, un durcissement dudit matériau dentaire, déterminé par recouvrance élastique selon ISO 4823 (édition 1992), dans un délai inférieur ou égal à 30 minutes, de préférence inférieur ou égal à 15 minutes, dans le cas d'une masse de duplication, et inférieur ou égal à 15 minutes, avec une préférence particulière inférieur ou égal à 10 min., avec une préférence toute particulière inférieur ou égal à 7 min., et avec la plus grande préférence inférieur ou égal à 6 min. dans le cas d'une masse pour empreintes dentaires.

5. Matériau dentaire selon l'une des revendications précédentes, **caractérisé en ce que** l'au moins une base de catalyseur b) est choisie dans le groupe constitué de 1,1,3,3-tétraméthylguanidine (TMG), 1,8-diazabicyclo[5.4.0]undéc-7-ène (DBU), 1,5-diazabicyclo[4.3.0]non-5-ène (DBN), tert.-butyliminotris-(diméthylamino)-phosphorane, tert.-butyliminotri(pyrrolidino)phosphorane, tert.-octylimino-tris-(diméthylamino)phosphorane, 2-tert.-butylimino-2-diéthylamino-1,3-diméthylperhydro-1,3,2-diazaphosphorine, 2-tert.-butylimino-2-diéthylamino-1,3-diméthylperhydro-1,3,2-diazaphosphorine sur polystyrène, 1-tert.-butyl-2,2,4,4,4-pentakis-(diéthylamino)-2Λ5,4Λ5-caténadi(phosphazène), 1-éthyl-2,2,4,4,4-pentakis-(diéthylamino)-2Λ5,4Λ5-caténadi(phosphazène), 1-tert.-butyl-4,4,4-tris-(diméthylamino)-2,2-bis-[tris-(diméthylamino)-phosphoranylidène-amino]-2Λ⁵,4Λ⁵-caténadi(phosphazène), 1-tert.-octyl-4,4,4-tris-(diméthylamino)-2,2-bis-[tris-(diméthylamino)-phosphoranylidène-amino]-2Λ⁵,4Λ⁵-caténadi(phosphazène), 2,8,9-triisobutyl-2,5,8,9-tetraaza-1-phosphabicyclo[3.3.3]undecane, 2,8,9-triisopropyl-2,5,8,9-tetraaza-1-phosphabicyclo[3.3.3]undécane, 2,8,9-triméthyl-2,5,8,9-tetraaza-1-phosphabicyclo[3.3.3]undecane, 2-tert.-butyl-1,1,3,3-tétraméthylguanidine, 1,8-bis-(tétraméthylguanidino)naphthalène (TMGN), 1,5,7-triazabicyclo(4.4.0)déc-5-ène, 7-méthyl-1,5,7-triazabicyclo(4.4.0)déc-5-ène, 1,5-diazabicyclo[4.3.0)non-5-ène et de 3,3,6,9,9-pentaméthyl-2,10-diazabicyclo-(4.4.0)déc-1-ène.

6. Matériau dentaire selon l'une des revendications précédentes, **caractérisé en ce qu'**il contient en outre au moins un sel de tampon e) qui est préférentiellement choisi dans le groupe constitué de bicarbonates de métaux alcalins, d'hydrogénophosphates dialcalins, de tris-(hydroxyméthyl)aminométhane, de sels monoalcalins d'acide phtalique, de sels de monotétraméthylammonium d'acide phtalique, de sels d'ammonium d'amines, d'amines cycliques, d'amides et amides cycliques, d'acide N-(2-acétamido)-2-aminoéthane-sulfonique, d'acide N-(2-acétamido)iminodiacétique, de β-alanine, d'acide formique (98 à 100 %), de 2-amino-2-méthyl-1-propanol, de barbital, de barbital-sodium, d'acide succinique, d'acide N,N-bis-(2-hydroxyéthyl)-2-aminoéthansulfonique, de N,N-bis-(2-hydroxyéthyl)-glycine, de 2,2-bis-(hydroxyéthyl)-(iminotris)-(hydroxyaméthyl)méthane, d'acide borique, de CHAPS (3-[(3-cholamidopropyl)-diméthylammonio]propanesulfonate), de monohydrate d'acide citrique, d'acide 2-(cyclohexylamino)-éthansulfonique, de diéthanolamine, d'acide acétique (acide acétique glacial ; 100 %), de glycine, de glycylglycine, d'acide 2-[4-2-hydroxyéthyl)-1-pipérazinyl]-éthansulfonique, de sel de sodium d'acide 2-[4-[2-hydroxyéthyl)-1-pipérazinyl]-éthansulfonique, d'acide 3-[4-(2-hydroxyéthyl)-1-pipérazinyl]-propanesulfonique, d'imidazole, d'hydrogénophosphate de potassium, de trihydrate d'hydrogénophosphate dipotassique, d'hydrogénophtalate de potassium, de 1-méthylimidazole, de monohydrate d'acide 2-morpholinoéthansulfonique, d'acide 3-morpholinopropansulfonique, de trihydrate d'acétate de sodium, de carbonate de sodium, de dihydrate de citrate trisodique, de monohydrate d'hydrogénophosphate de sodium, de décahydrate de diphosphate tétrasodique, de bicarbonate de sodium, de dihydrate d'hydrogénophosphate disodique, d'oxalate disodique, de décahydrate de tétraborate disodique, d'acide pipérazino-1,4-bis-(2-éthansulfonique), de sel de monosodium d'acide pipérazino-1,4-bis-(2-éthansulfonique), de triéthanolamine, d'hydrochlorure de triéthanolamine, de 2,4,6-triméthylpyridine, de tris-(hydroxyméthyl)-aminométhane, d'hydrochlorure de tris-(hydroxyméthyl)-aminométhane, d'acide N-[tris-(hydroxyméthyl)-méthyl]-2-aminoéthanesulfonique et d'acide N-[tris-(hydroxyméthyl)-méthyl]-3-aminopropanesulfonique.

7. Matériau dentaire selon l'une des revendications précédentes, **caractérisé en ce que** il contient en outre au moins un absorbant d'humidité d), s'agissant préférentiellement de vinyltriméthoxysilane, N-triméthoxysilylméthyl-O-méthylcarbamate et/ou d'un composé répondant à la formule suivante: 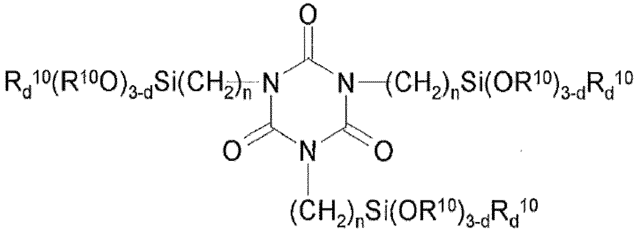 dans laquelle n = 1 à 6, de préférence n = 1 à 3, avec une préférence particulière n = 1,
d = 0 ou 1, et
R¹⁰ = un radical alkyl en C₁-C₃₀ de nature linéaire ou ramifiée, dans lequel les atomes d'hydrogène peuvent partiellement être substitués par des atomes d'halogène, des radicaux OH-, NH₂, NO₂ ou bien par d'autres radicaux alkyle en C₁-C₆.

8. Matériau dentaire selon l'une des revendications précédentes, **caractérisé en ce qu'**il contient en outre au moins un agent formateur de pâte h) qui est préférentiellement choisi dans le groupe constitué de polyéthers, de polyvinylpyrrolidones, de polyuréthanes, de polyesters, de cires, de vaseline, d'huiles de paraffine, d'huile de silicone, de polyalcools, de propylèneglycols, de polypropylèneglycols, d'éthylèneglycols, de polyéthylèneglycols, de produits de copolymérisation de N-vinylpyrrolidone et d'acétate de vinyle, de carboxyméthyl-, méthyl-, hydroxyéthyl-, hydroxypropylcellulose, de polysaccharides et d'acides poly(méth)acryliques.

9. Matériau dentaire selon l'une des revendications précédentes, **caractérisé en ce qu'**il contient en outre au moins un agent stabilisateur u) qui est préférentiellement choisi dans le groupe constitué de triméthyldihydroquinoline sous une forme polymère, de dérivés diphényliques, de phénothiazine, de phényl-α-naphtylamine, de 4,4'-méthylène-bis-2,6-di-tert.-butylphénol, de butylhydroxytoluène, de butylhydroxyanisole (BHA) et de méthoxyphénol (hydroxyanisole).

10. Matériau dentaire selon l'une des revendications précédentes, **caractérisé en ce qu'**il ne contient, outre une ou plusieurs bases selon l'une des revendications 1 à 6, aucun autre catalyseur, notamment pas de composés organométalliques, pas de sels de carboxylates de métaux lourds, pas d'amines ayant une valeur pk_{BH+} inférieure à 20, et pas d'acides libres.

11. Matériau dentaire selon l'une des revendications précédentes, **caractérisé en ce qu'**il contient, en tant que catalyseur d), 0,001 à 1,0 mmol/g, avec une préférence particulière 0,001 à 0,5, avec une préférence toute particulière 0,001 à 0,1 mmol/g et, avec la plus grande préférence, 0,005 à 0,05 mmol/g d'au moins une base organique, par rapport au mélange dans son intégralité.

12. Matériau dentaire selon la revendication 13, **caractérisé en ce que** n est égal à 1.

13. Matériau dentaire selon l'une des revendications précédentes, **caractérisé en ce que** l'au moins un polyéther a) contient une unité structurelle choisie dans le groupe constitué de polytétrahydrofurane, polyéthylèneglycol, polypropylèneglycol et de leurs produits de copolymérisation et leurs mélanges.

14. Mélange pouvant être obtenu en mélangeant les composants A et B dudit matériau dentaire à deux composants selon l'une des revendications 2 à 13, **caractérisé en ce que** le composant de base A est mélangé avec le composant catalytique B dans un rapport compris entre 1 : 1 et 20 : 1, avec une préférence particulière compris entre 1 : 1 et 10 : 1, et avec une préférence toute particulière égal à 1 : 1, 2 : 1, 4 : 1, 5 : 1 et 10 : 1.

15. Utilisation d'un matériau dentaire selon l'une des revendications 1 à 14 dans les soins dentaires et/ou dans la technique dentaire.
